(19) Europäisches Patentamt
European Patent Office
Office européen des brevets

(11) **EP 4 193 997 A1**

(12) **EUROPEAN PATENT APPLICATION**
published in accordance with Art. 153(4) EPC


(43) Date of publication:
**14.06.2023 Bulletin 2023/24**

(21) Application number: **21855318.8**

(22) Date of filing: **14.07.2021**

(51) International Patent Classification (IPC):
***A61K 31/517*** *(2006.01)* ***A61K 31/428*** *(2006.01)*
***A61K 31/138*** *(2006.01)* ***A61P 13/12*** *(2006.01)*
***A61P 3/10*** *(2006.01)*

(52) Cooperative Patent Classification (CPC):
**A61K 31/138; A61K 31/428; A61K 31/517; A61P 3/10; A61P 13/12**

(86) International application number:
**PCT/CN2021/106355**

(87) International publication number:
**WO 2022/033265 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.08.2020 CN 202010794864**



(71) Applicant: **Chengdu Wending Technology Development Co., Ltd.**
**Chengdu, Sichuan 610041 (CN)**

(72) Inventors:
- **ZENG, Wen**
**Chengdu, Sichuan 610094 (CN)**
- **YANG, Zhenyan**
**Chengdu, Sichuan 610094 (CN)**

(74) Representative: **Slingsby Partners LLP**
**1 Kingsway**
**London WC2B 6AN (GB)**


(54) **METHOD FOR TREATING CHRONIC KIDNEY DISEASE AND PHARMACEUTICAL COMPOSITION**


(57) The present application refers to a method and pharmaceutical composition for treating chronic kidney disease. The method and pharmaceutical composition according to the present application relate to the combined use of doxazosin, pramipexole and metoprolol (or a pharmaceutically acceptable salt thereof), which can significantly improve the chronic kidney disease, significantly reduce urinary albumin excretion rate or UACR, significantly improve the glomerular filtration rate eGFR, and effectively delay the progression of renal dysfunction to end-stage renal failure.


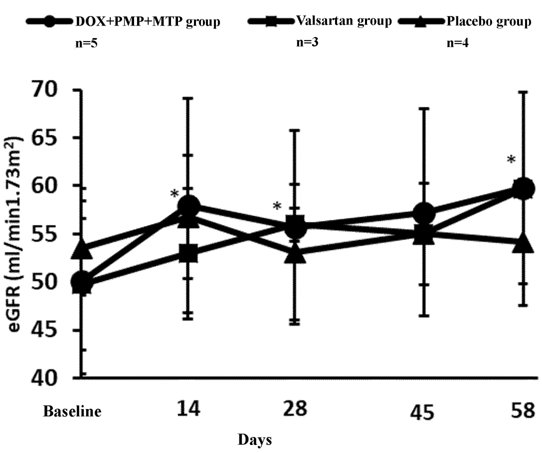


Fig. 3

EP 4 193 997 A1

## Description

### TECHNICAL FIELD

The present application relates to the field of disease treatment and medicine, in particular to the treatment of chronic kidney disease, especially diabetic kidney disease.

### BACKGROUND

Chronic kidney disease (CKD) is a disease that seriously affects human health, especially the health of the elderly. According to statistics, the prevalence of CKD in American adults is as high as 11.3%, while the prevalence of CKD in China is estimated to be about 10%. Diabetes and hypertension are considered to be the main predisposing factors of CKD.

Diabetes is one of the common diseases affecting human health. In 2015, there were nearly 420 million diabetic patients in the world. It is estimated that the number of diabetic patients will reach 640 million by 2040, and it is estimated that 20% to 40% of diabetic patients may develop diabetic kidney disease. Diabetic kidney disease (DKD) is a common complication of diabetes, also known as DN (Diabetic Nephropathy) in the literature. Diabetic kidney disease is a lesion with abnormal structure and function of the kidney caused by chronic diabetic microangiopathy. The clinical manifestations of diabetic kidney disease mainly include hypertension, proteinuria, and edema. The pathological manifestations of diabetic kidney disease include glomerular vascular damage and nodular lesions caused by sclerosis, which may in turn lead to abnormal renal function and persistent proteinuria and eventually lead to renal failure and End-Stage Renal Disease (ESRD) with a high mortality rate.

DKD is a complex disease involving both environmental and genetic factors. The reported mechanism of occurrence and development is very complex, and involves pathological changes, including oxidative stress, inflammation, renal function injury, renal interstitial fibrosis, hemodynamic changes, genetic factors, etc. Among them, oxidative stress plays an important role in the pathogenesis and is considered to be closely related to the occurrence and development of DKD.

Current management strategies for DKD are mainly to achieve optimal glycemic and blood pressure control, correct dyslipidemia, reverse insulin resistance, and reduce proteinuria (using angiotensin-converting enzyme inhibitors and angiotensin receptor blockers), thereby delaying the deterioration of renal function and reducing cardiovascular risk events. Inhibition of renin-angiotensin-aldosterone system (RAAS) activation by using angiotensin-converting enzyme inhibitors (ACEIs) and/or angiotensin receptor blockers (ARBs) is currently the main method for the treatment of DKD. Valsartan is a commonly used ARB in clinical practice. By improving the selective permeability of glomeruli while keeping the radius of glomerular filtration pores unchanged, it can continuously reduce urinary albumin without causing a decrease in the estimated glomerular filtration rate (eGFR). Valsartan is a commonly used drug for the treatment of DKD. However, the existing treatments and drugs are still less effective in preventing and controlling the progression of diabetic kidney disease. Studies have shown that ARBs do not increase the risk of myocardial infarction, and can further reduce the risk of heart failure and stroke; however, it is also found that ARBs cannot reduce the all-cause death of the included patients. Moreover, for diabetic patients with normal blood pressure and without proteinuria, the use of ACEI or ARB drugs cannot prevent the occurrence of microalbuminuria.

Therefore, there is an urgent clinical need for the development of new drugs that can effectively treat CKD and/or DKD, and/or effectively delay the progression of CKD/DKD to ESRD.

### SUMMARY

Therefore, objective of the present application is to provide a better or an alternative treatment method and medicine for treating chronic kidney disease, especially diabetic kidney disease.

The inventors have surprisingly found that the combination of doxazosin (DOX), pramipexole (PMP) and metoprolol (MTP) has significant curative effect in the treatment of chronic kidney disease, especially diabetic kidney disease.

Therefore, the first aspect of the present application is a pharmaceutical composition for treating chronic kidney disease, comprising doxazosin (or a pharmaceutically acceptable salt thereof), pramipexole (or a pharmaceutically acceptable salt thereof), and metoprolol (or a pharmaceutically acceptable salt thereof) as active ingredients.

A second aspect of the present application relates to use of the combination of doxazosin (or a pharmaceutically acceptable salt thereof), pramipexole (or a pharmaceutically acceptable salt thereof) and metoprolol (or a pharmaceutically acceptable salt thereof) in the preparation of a pharmaceutical composition for treatment of chronic kidney disease, especially diabetic kidney disease, in a subject in need thereof.

A third aspect of the present application relates to use of the combination of doxazosin (or a pharmaceutically acceptable salt thereof), pramipexole (or a pharmaceutically acceptable salt thereof) and metoprolol (or a pharmaceutically acceptable salt thereof) in treatment of chronic kidney disease, especially diabetic kidney disease, in a subject in need thereof.

A fourth aspect of the present application relates to a method for treating chronic kidney disease, especially diabetic kidney disease, the method comprising administering a therapeutically effective amount of doxazosin (or a pharmaceutically acceptable salt thereof), pramipexole (or a pharmaceutically acceptable salt thereof) and metoprolol (or a pharmaceutically acceptable salt thereof) to a subject in need.

By using rhesus monkeys with spontaneous chronic diabetic kidney disease as animal models to simulate human DKD patients, the inventors have unexpectedly found that the combination of doxazosin, pramipexole and metoprolol can significantly improve proteinuria and glomerular filtration rate, thereby controlling or delaying the progression of DKD to end-stage renal disease (ESRD), and the combination is safe as no drug-related adverse event was observed during the administration period. Therefore, the drug combination in the present application is a new therapy for chronic kidney disease, especially diabetic kidney disease.

## BRIEF DESCRIPTION OF THE FIGURES

Hereafter, the particular embodiments of the present application will be described in detail with reference to the accompanying figures.

Fig. 1 shows the comparison of changes in albuminuria (UACR, urine albumin-creatinine rate) of rhesus monkeys in each group on day 30 (D30) of drug administration (**, $p<0.01$, compared with the placebo group; *, $p<0.05$, compared with the placebo group).

Fig. 2 shows the comparison of changes in albuminuria (UACR, urine albumin-creatinine rate) of rhesus monkeys in each group on day 58 (D58) of drug administration (**, $p<0.01$, compared with the placebo group; *, $p<0.05$, compared with the placebo group).

Fig. 3 shows the changes in eGFR of rhesus monkeys in each group after administration (eGFR selected in each group: 30 to 59 ml/min/1.73$m^2$ for analysis; DOX+PMP+MTP 1# experimental group and DOX+PMP+MTP 2# experimental group were combined and analyzed as one group; *, $p<0.05$ for DOX+PMP+MTP group, compared with the placebo group).

## DETAILED DESCRIPTION

The present application relates to combinations of multiple GPCR drugs and corresponding methods of combination therapy.

G protein (guanine nucleotide-binding protein) coupled receptors (GPCRs) are a large class of transmembrane proteins that regulate intracellular signaling and are required for cellular homeostasis. Studies in literatures have shown that the GPCR signaling pathway is related to diabetes-induced superoxide generation (Du, Y, et al., Adrenergic and serotonin receptors affect retinal superoxide generation in diabetic mice: Relationship to capillary degeneration and permeability. 2015. 29(5): p. 2194). Although oxidative stress is an important mechanism in the development of DKD and GPCR drugs that regulate diabetes-induced superoxide production may theoretically have a certain impact on the development of DKD, existing research is limited to the pathogenesis level. The therapeutic effect of GPCR drugs on DKD is still unknown and need to be studied. In addition, GPCR drugs cover hundreds of different substances, and it is also difficult to screen out clinically effective drugs for DKD.

Moreover, the current animal models for studying diabetic kidney disease are mostly rodent animals, such as drug-induced mouse models, spontaneous db/db mice, ob/ob mice, Agout mutant mice, New Zealand obese mice, and transgenic mouse models, etc. However, since the pathogenesis of diabetic kidney disease is complex and involves many factors, the damage of kidney function in these animal models is difficult to accurately reflect the course of DKD in patients, and these animal models are not suitable for evaluating the effects of treatment of disease. Non-human primates are very similar to humans in terms of physiology, biochemistry, and systems biology. Many studies have reported that the disease characteristics of rhesus monkeys with spontaneous chronic diabetic kidney disease are very similar to those of clinical DKD patients, and they also show moderate-to-heavy proteinuria, a decreased eGFR and other characteristics, so rhesus monkeys with spontaneous chronic diabetic kidney disease provide an important research method for CKD/DKD research and new drug development (Najafian, B., et al., Glomerulopathy in spontaneously obese rhesus monkeys with type 2 diabetes: a stereological study. Diabetes Metab Res Rev, 2011. 27(4): p. 341-7. Liang Y, Yang Z, et al. Diabetic Kidney Disease (DKD) in Nonhuman Primates (NHPs) is Comparable to Humans for Glomerular Filtration Rate (GFR), Histology and High Risk Factors. ADA2017).

Considering the limitations of rodent animal models in the study of human diabetic kidney disease, the inventors have used rhesus monkeys with spontaneous chronic diabetic kidney disease as animal models to simulate human patients and conducted a large number of meticulous research and extensive screening of GPCR drugs. As a result, it

is unexpectedly found that some GPCR drugs have a significant effect on DKD when they are combined together. Specifically, it has been found that the combination of doxazosin, pramipexole, and metoprolol can significantly reduce proteinuria (UACR) of rhesus monkey animal model with moderate to severe proteinuria in the CKD-EPI stage G3, significantly improve glomerular filtration rate eGFR and inhibit the development of DKD to end-stage renal disease (ESRD), and its efficacy is comparable to that of valsartan, which is a commonly used first-line clinical drug. Based on this, the present application discloses a pharmaceutical composition for treating chronic kidney disease, especially DKD, and its medical use.

## I. Pharmaceutical composition

The first aspect of the present application provides a pharmaceutical composition for treating chronic kidney disease and especially diabetic kidney disease, comprising doxazosin (or a pharmaceutically acceptable salt thereof), pramipexole (or a pharmaceutically acceptable salt thereof), and metoprolol (or a pharmaceutically acceptable salt thereof) as active ingredients.

Certain terms are used in the specification, embodiments and claims of this application. Unless defined otherwise, all technical and scientific terms used herein have the same meaning as commonly understood by one of ordinary skill in the art to which this application belongs.

The terms "comprise", "comprising", "include", "including", "have" and "having" are used in an inclusive and open sense, which means that additional elements may be included other than those specified. The terms "such as" and "for example" used herein are non-limiting and are for the purpose of illustration only. The terms "including" and "including but not limited to" are used interchangeably.

Unless the context clearly indicates otherwise, the term "or", as used herein, shall be understood to mean "and/or".

The term "treating" refers to inhibiting a disease, disorder and/or symptom in a subject, e.g., impeding its progression; and relieving the disease, disorder and/or symptom, e.g., causing the regression of the disease, disorder and/or symptom. Treating a disease or disorder includes ameliorating at least one symptom of a particular disease or disorder, even if the underlying pathophysiology is not affected. In particular, "treatment" in this application also covers the meaning of reducing the risk of a certain disease by administration of a medication, that is, "treatment" includes not only prevention before the onset, but also remission, inhibition and cure of the disease after the onset.

The term "pharmaceutical composition" refers to a combination of substances having a specific medical or biological use, and is generally expected to have a therapeutic or prophylactic effect on a specific disease after being administered to a subject. The pharmaceutical composition may contain only the prescribed active ingredient (biologically active substance), or may be provided together with conventional pharmaceutically acceptable carriers for various purposes. The term "composition" in this application should be interpreted broadly. As an implementation of the pharmaceutical composition according to the present application, for example, it can be provided in the form of an indistinguishable mixture of the specified active ingredients by mixing the ingredients (and optional pharmaceutically acceptable carriers) together. As another implementation of the pharmaceutical composition according to the present application, the "pharmaceutical composition" of the present application may also be provided by individually packaging each specified active ingredient into small portion in a small independent package, and then combining and accommodating these small independent packages in a larger container.

The terms "active ingredient" and "biologically active substance" refer to molecules and other agents that are biologically, physiologically or pharmaceutically active for the treatment of a disease or condition (e.g., diabetic kidney disease) in a patient or subject. This term is used relative to the terms such as "pharmaceutically acceptable carrier", "excipient", "auxiliary". An "active ingredient" (or "biologically active substance") includes, but is not limited to, pharmaceutically acceptable salts and prodrugs thereof. Such agents may be acids, bases, or salts; they may be neutral molecules, polar molecules, or molecular complexes capable of hydrogen bonding; they may be prodrugs in the form of ethers, esters, amides and the like that are biologically activated when administered into a patient or subject.

The term "doxazosin" as used herein refers to the following molecule having the English name of Doxazosin ("DOX" for short) and the IUPAC name of (RS)-2-[4-(2,3-dihydro-1,4-benzodioxine-2-carbonyl)piperazin-1-yl]-6,7-dimethoxyquinazolin-4-amine (molecular formula $C_{23}H_{25}N_5O_5$, molecular weight: 451.475 g/mol):

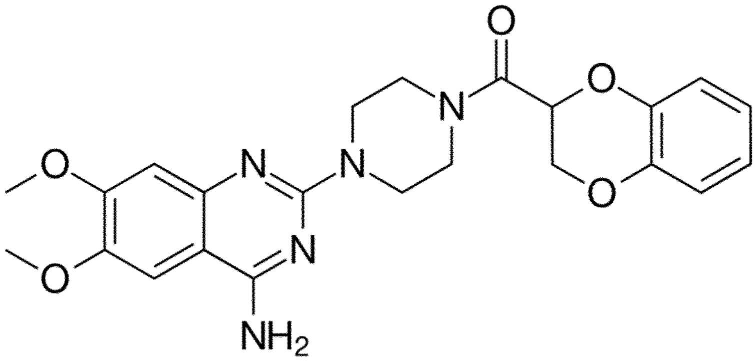

The term "doxazosin" also covers its isotope-labeled compounds, or their optical isomers, geometric isomers, tautomers or isomer mixtures, or their prodrugs (i.e. the above-mentioned molecular compounds obtained through *in vivo* reactions).

Doxazosin, a marketed drug approved by major pharmaceutical regulatory agencies (such as the FDA), is a selective $\alpha$1-receptor antagonist that inhibits the binding of norepinephrine (released from sympathetic nerve endings) with the $\alpha$-1 receptor on vascular smooth muscle cell membranes, and is often used for the treatment of essential hypertension.

The pharmaceutical composition according to the present application may contain doxazosin or a pharmaceutically acceptable salt thereof as an active ingredient. Most of the commercially available doxazosin drugs are in salt form, especially its mesylate salt, for example doxazosin mesylate extended release tablets (Cardura) available from the Pfizer Inc. and doxazosin mesylate tablets available from Hangzhou Conba Pharmaceutical Co., Ltd.

The term "pramipexole" as used herein refers to the following molecule having the English name of Pramipexole ("PMP" for short) and the IUPAC name of (S)-N6-propyl-4,5,6,7-tetrahydro-1,3-benzothiazole-2,6-diamine (molecular formula $C_{10}H_{17}N_3S$, molecular weight: 211.324 g/mol):

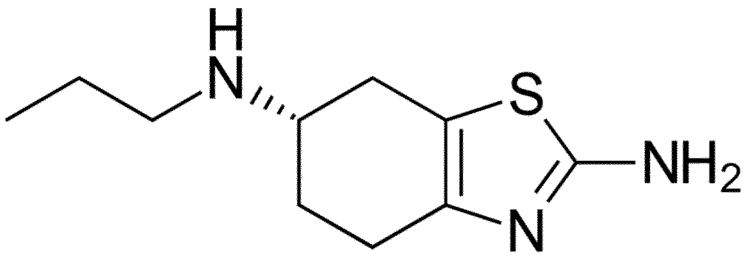

The term "pramipexole" also covers its isotope-labeled compounds, or their optical isomers, geometric isomers, tautomers or isomer mixtures, or their prodrugs (i.e. compounds that gives the above-mentioned molecule through *in vivo* reactions).

Pramipexole, a marketed drug approved by major medical regulatory agencies (such as the FDA), is an antihistamine and acts as a dopamine receptor D2/D3 agonist. It is mainly used clinically for the treatment of Parkinson's disease, alone (without levodopa) or in combination with levodopa. In the literature, pramipexole is sometimes referred to as "milapa" or "Mirapex", "Mirapexin", "Sifrol" and the like.

The pharmaceutical composition according to the present application may contain pramipexole or a pharmaceutically acceptable salt thereof as an active ingredient. Most of the commercially available pramipexole is in the form of salt, especially its hydrochloride, for example, pramipexole hydrochloride tablets (Sifrol) available from Boehringer Ingelheim pharmaceutical company.

The term "metoprolol" as used herein refers to the following molecules with the English name of Metoprolol ("MTP" for short) and IUPAC name of (RS)-1-[4-(2-methoxyethyl)phenoxy]-3-[(propan-2-yl)amino]propan-2-ol (molecular formula $C_{15}H_{25}NO_3$, molecular weight 267.37 g/mol):

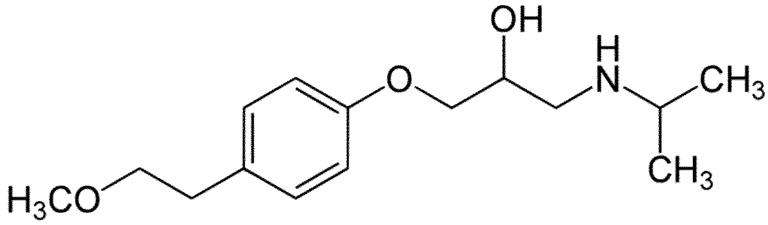

The term "metoprolol" also covers its isotope-labeled compounds, or their optical isomers, geometric isomers, tautomers or isomer mixtures, or their prodrugs (i.e. compounds that gives the above-mentioned molecule through *in vivo* reactions).

Metoprolol, a marketed drug approved by major pharmaceutical regulatory agencies (such as the FDA), is a selective adrenaline β1 receptor blocker and commonly used for the treatment of high blood pressure and angina pectoris. The pharmaceutical composition according to the present application may contain metoprolol or a pharmaceutically acceptable salt thereof as an active ingredient. Most of commercially available metoprolol are in salt forms, especially tartrates, such as metoprolol tartrate tablets (Betaloc) available from AstraZeneca Pharmaceuticals Ltd., and metoprolol tartrate controlled release tablets available from Guangzhou Baiyunshan Tianxin Pharmaceutical Co., Ltd. (LiJunning) and the like.

The active ingredients (doxazosin, pramipexole, metoprolol) contained in the pharmaceutical composition according to the present application may be replaced by their pharmaceutically acceptable salts. The term "pharmaceutically acceptable salt" of a compound refers to a salt that is pharmaceutically acceptable and possesses the desired pharmacological activity of the parent compound. A pharmaceutically acceptable salt as used herein is a salt formed with a pharmaceutically acceptable acid or base. Pharmaceutically acceptable acids include, but are not limited to, inorganic acids such as hydrochloric acid, sulfuric acid, phosphoric acid, pyrophosphoric acid, hydrobromic acid, or nitric acid; and organic acids such as citric acid, fumaric acid, maleic acid, malic acid, ascorbic acid, succinic acid, tartaric acid, benzoic acid, acetic acid, methanesulfonic acid, ethanesulfonic acid, salicylic acid, stearic acid, benzenesulfonic acid or p-toluenesulfonic acid. Pharmaceutically acceptable bases include hydroxides of alkali metal (such as sodium or potassium), and alkaline earth metal (such as calcium or magnesium); and organic bases such as alkyl amines, aryl amines or heterocyclic amines. For the avoidance of doubt, the "pharmaceutically acceptable salt" of an active ingredient described in this application also includes pharmaceutically acceptable salts formed by isotopically labeled compounds of the active ingredient, or optical isomers, geometric isomers, tautomers or mixtures of isomers, or prodrugs thereof.

Pharmaceutically acceptable salts can be synthesized from the parent compound which contains a basic or acidic moiety by conventional chemical methods. Generally, these salts can be prepared by reacting the free acid or base forms of these compounds with a stoichiometric amount of the appropriate base or acid in water or in an organic solvent, or in a mixture of the two; generally, non-aqueous media like diethyl ether, ethyl acetate, ethanol, isopropanol, or acetonitrile. A list of salts can be found in Remington's Pharmaceutical Sciences 18th Edition (Mack Publishing Company, 1990). For example, salts may include, but are not limited to, hydrochloride, tartrate, methanesulfonate, and the like of the compounds described herein.

It should be understood that all references to various active ingredients or pharmaceutically acceptable salts include solvent addition forms (solvates such as hydrates, ethanol solvates, acetone solvates, etc.) or various crystal forms (such as amorphous forms, polymorphous forms, etc.) of the same active ingredients or salts.

The amounts of doxazosin (or a pharmaceutically acceptable salt thereof), pramipexole (or a pharmaceutically acceptable salt thereof), and metoprolol (or a pharmaceutically acceptable salt thereof) in the pharmaceutical composition according to the present application can be adjusted according to actual needs. For example, the content or ratio of each drug in the pharmaceutical composition can be changed according to the administration mode (oral or injection, etc.) of the pharmaceutical composition.

In some embodiments, the pharmaceutical composition according to the present application usually comprises 0.5 to 45 parts by weight (preferably 1 to 10 parts by weight) of pramipexole or a pharmaceutically acceptable salt thereof, 5 to 160 parts by weight (preferably 5 parts by weight to 80 parts by weight) of doxazosin or a pharmaceutically acceptable salt thereof, and 50 parts by weight to 2000 parts by weight (preferably 100 parts by weight to 1000 parts by weight) of metoprolol or a pharmaceutically acceptable salt thereof.

In the pharmaceutical composition according to the present application, the amount of pramipexole or a pharmaceutically acceptable salt thereof is usually 0.5 to 45 parts by weight, for example, it can be 0.5 parts by weight to 42 parts by weight, 0.5 parts by weight to 40 parts by weight, 0.5 parts by weight to 35 parts by weight, 0.5 parts by weight to 30 parts by weight, 0.5 parts by weight to 25 parts by weight, 0.5 parts by weight to 20 parts by weight, 0.5 parts by weight to 15 parts by weight, 0.5 parts by weight to 10 parts by weight, 1 part by weight to 45 parts by weight, 1 part by weight to 42 parts by weight, 1 part by weight to 40 parts by weight, 1 part by weight to 35 parts by weight, 1 part by weight to 30 parts by weight, 1 part by weight to 25 parts by weight, 1 part by weight to 20 parts by weight, 1 part by

weight to 15 parts by weight, 1 part by weight to 10 parts by weight, 2 parts by weight to 45 parts by weight, 2 parts by weight to 40 parts by weight, 2 parts by weight to 35 parts by weight, 2 parts by weight to 30 parts by weight, 2 parts by weight to 25 parts by weight, 2 parts by weight to 20 parts by weight, 2 parts by weight to 15 parts by weight, 2 parts by weight to 10 parts by weight, 5 parts by weight to 45 parts by weight, 5 parts by weight to 40 parts by weight, 5 parts by weight to 35 parts by weight, 5 parts by weight to 30 parts by weight, 5 parts by weight to 25 parts by weight, 5 parts by weight to 20 parts by weight, 5 parts by weight to 15 parts by weight, 5 parts by weight to 10 parts by weight, 10 parts by weight to 40 parts by weight, 10 parts by weight to 30 parts by weight, 10 parts by weight to 25 parts by weight, and the like. In a preferred embodiment of the present application, the amount of pramipexole or a pharmaceutically acceptable salt thereof is 1 to 10 parts by weight.

In the pharmaceutical composition according to the present application, the amount of doxazosin or a pharmaceutically acceptable salt thereof is usually 5 parts by weight to 160 parts by weight, for example, it can be 5 parts by weight to 150 parts by weight, 5 parts by weight to 130 parts by weight, 5 parts by weight to 120 parts by weight, 5 parts by weight to 100 parts by weight, 5 parts by weight to 80 parts by weight, 5 parts by weight to 60 parts by weight, 5 parts by weight 50 parts by weight, 5 parts by weight to 40 parts by weight, 10 parts by weight to 150 parts by weight, 10 parts by weight to 130 parts by weight, 10 parts by weight to 120 parts by weight, 10 parts by weight to 100 parts by weight, 10 parts by weight to 80 parts by weight, 10 parts by weight to 60 parts by weight, 10 parts by weight to 50 parts by weight, 10 parts by weight to 40 parts by weight, 15 parts by weight to 160 parts by weight, 15 parts by weight to 150 parts by weight, 15 parts by weight to 130 parts by weight, 15 parts by weight to 120 parts by weight, 15 parts by weight to 100 parts by weight, 15 parts by weight to 80 parts by weight, 15 parts by weight to 60 parts by weight, 15 parts by weight to 50 parts by weight, 15 parts by weight to 40 parts by weight, 20 parts by weight to 160 parts by weight, 20 parts by weight to 150 parts by weight, 20 parts by weight to 130 parts by weight, 20 parts by weight to 120 parts by weight, 20 parts by weight to 100 parts by weight, 20 parts by weight to 80 parts by weight, 20 parts by weight to 60 parts by weight, 20 parts by weight 50 parts by weight, 20 parts by weight to 40 parts by weight and the like. In a preferred embodiment of the present application, the amount of doxazosin or a pharmaceutically acceptable salt thereof is 5 to 80 parts by weight.

In the pharmaceutical composition according to the present application, the amount of metoprolol or a pharmaceutically acceptable salt thereof is usually 50 parts by weight to 2000 parts by weight, for example, it can be 50 parts by weight to 1800 parts by weight, 50 parts by weight to 1600 parts by weight, 50 parts by weight to 1500 parts by weight, 50 parts by weight to 1300 parts by weight, 50 parts by weight to 1200 parts by weight, 50 parts by weight to 1000 parts by weight, 50 parts by weight to 800 parts by weight, 50 parts by weight to 600 parts by weight, 50 parts by weight to 500 parts by weight, 50 parts by weight to 400 parts by weight, 100 parts by weight to 1800 parts by weight, 100 parts by weight to 1600 parts by weight, 100 parts by weight to 1500 parts by weight, 100 parts by weight to 1300 parts by weight, 100 parts by weight to 1200 parts by weight, 100 parts by weight to 1000 parts by weight, 100 parts by weight to 800 parts by weight, 100 parts by weight to 600 parts by weight, 100 parts by weight to 500 parts by weight, 100 parts by weight to 400 parts by weight, 150 parts by weight to 2000 parts by weight, 150 parts by weight to 1800 parts by weight, 150 parts by weight to 1600 parts by weight, 150 parts by weight to 1500 parts by weight, 150 parts by weight to 1300 parts by weight, 150 parts by weight to 1200 parts by weight, 150 parts by weight to 1000 parts by weight, 150 parts by weight to 800 parts by weight, 150 parts by weight to 600 parts by weight, 200 parts by weight to 2000 parts by weight, 200 parts by weight to 1800 parts by weight, 200 parts by weight to 1600 parts by weight, 200 parts by weight to 1500 parts by weight, 200 parts by weight to 1300 parts by weight, 200 parts by weight to 1200 parts by weight, 200 parts by weight to 1000 parts by weight, 200 parts by weight to 800 parts by weight, 200 parts by weight to 600 parts by weight, and the like. In a preferred embodiment of the present application, the amount of doxazosin or a pharmaceutically acceptable salt thereof is 100 to 1000 parts by weight.

In the pharmaceutical composition according to the present application, if the weight of doxazosin or a pharmaceutically acceptable salt thereof is used as the reference (=1), weight ratio of metoprolol or a pharmaceutically acceptable salt thereof to doxazosin or a pharmaceutically acceptable salt thereof is generally in the range of 0.3:1 to 400:1. For example, it can be greater than or equal to 0.4:1, 0.5:1, 0.6:1, 0.7:1, 0.8:1, 0.9:1, 1:1, 1.5:1, 2:1, 3:1, 4:1, 5:1, 10:1, 12.5:1 or 15:1, preferably greater than or equal to 1:1, 1.5:1, 2:1, 3:1, 4:1, 5:1, 10:1, 12.5:1 or 15:1, and can be less than or equal to 350:1, 300:1, 250:1, 200:1, 150:1, 100:1, 90:1, 80:1, 70:1, 60:1, 50:1, 40:1, 30:1 or 20:1, preferably less than or equal to 100:1, 90:1, 80:1, 70:1, 60:1, 50:1, 40:1, 30:1 or 20:1. Likewise, if the weight of doxazosin or a pharmaceutically acceptable salt thereof is used as the reference (=1), weight ratio of pramipexole or a pharmaceutically acceptable salt thereof to doxazosin or a pharmaceutically acceptable salt thereof is generally in the range of 0.003:1 to 10:1. For example, it can be greater than or equal to 0.004:1, 0.005:1, 0.006:1, 0.007:1, 0.008:1, 0.009:1, 0.01:1, 0.015:1, 0.02:1, 0.03:1, 0.04:1, 0.05:1, 0.06:1, 0.07:1, 0.08:1, 0.1:1 or 0.2:1, preferably greater than or equal to 0.005:1, 0.006:1, 0.007:1, 0.008:1, 0.009:1, 0.01:1, 0.015:1, 0.02:1, 0.03:1, 0.04:1, 0.05:1, 0.06:1, 0.07:1, 0.08:1 or 0.1:1, and can be less than or equal to 9:1, 6:1, 5:1, 4:1, 3:1, 2:1, 1:1, 0.9:1, 0.6:1, 0.5:1, 0.4:1 or 0.3:1, preferably less than or equal to 1:1, 0.9:1, 0.6:1, 0.5:1 or 0.4:1.

II. Drug dosage form

In some embodiments, the pharmaceutical composition according to the present application can be provided in the form of a bulk drug (such as a homogeneous mixture or individual packaging of each ingredient). In some further embodiments, pharmaceutically acceptable carriers as needed can be added into the pharmaceutical composition according to the present application to formulate various pharmaceutical dosage forms (or preparations). For this purpose, various liquid or solid fillers, diluents, excipients, solvents or encapsulating materials can be used as the "pharmaceutically acceptable carriers". In this application, the phrase "pharmaceutically acceptable" means those compounds, compositions, polymers and other materials, within the scope of sound medical judgment, compatible with the other ingredients of the composition according to the present application and suitable for contact with tissues of human and animals without undue toxicity, irritation, allergic response or other problems or complications. In certain preferred embodiments, a pharmaceutically acceptable carrier is non-pyrogenic. Some examples of materials which may serve as pharmaceutically acceptable carriers include: (1) sugars, such as lactose, glucose, and sucrose; (2) starches, such as corn starch and potato starch; (3) cellulose and its derivatives, such as sodium carboxymethyl cellulose, ethyl cellulose, and cellulose acetate; (4) powdered tragacanth; (5) maltodextrin; (6) gelatin; (7) talc; (8) excipients, such as cocoa butter and suppository waxes; (9) oils such as peanut oil, cottonseed oil, sunflower oil, sesame oil, olive oil, corn oil, and soybean oil; (10) glycols, such as propylene glycol; (11) polyols, such as glycerin, sorbitol , mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethanol; (20) phosphate buffer solutions; (21) other non-toxic compatible substances employed in pharmaceutical preparations.

The pharmaceutical composition of the present application may be formulated as suitable dosage forms for oral, parenteral (including subcutaneous, muscular, intradermal and intravenous), bronchial, intravitreous injection or nasal administration as desire. Preferably, the pharmaceutical composition according to the present application is formulated into a dosage form (preparation) suitable for oral administration.

If a solid carrier is used, the preparation may be tableted, placed in a hard gelatin capsule in powder or pellet form, or presented in troche or lozenge form. The solid carrier may include conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, soft gelatin capsule, sterile vehicle for injection, an aqueous or non-aqueous liquid suspension, or may be a dry product for reconstitution with water or other suitable vehicle before use. Liquid formulations may contain conventional additives such as suspending agents, emulsifying agents, wetting agents, non-aqueous vehicles (including edible oils), preservatives and flavoring and/or coloring agents. For parenteral administration, the carrier will usually comprise sterile water, at least in large part, but saline solutions, glucose solutions and the like may also be utilized. Injectable suspensions may also be used, in which case conventional suspending agents may be employed. Conventional preservatives, buffering agents and the like may also be added to the parenteral dosage forms.

Dosage forms suitable for parenteral injection may include physiologically acceptable sterile aqueous or non-aqueous solutions, dispersions, suspensions or emulsions and sterile powders for sterile injectable solutions or dispersions. Examples of suitable aqueous and non-aqueous carriers, diluents, solvents include water, ethanol, polyols (propylene glycol, polyethylene glycol, glycerol, and the like), vegetable oils (such as olive oil), injectable organic esters (such as ethyl oleate), and suitable mixtures thereof,.

These pharmaceutical dosage forms may also contain various excipients, for example, preservatives, wetting agents, emulsifying agents and dispersing agents. Inhibition of the action of microorganisms can be ensured by various antibacterial and antifungal agents (for example, parabens, chlorobutanol, phenol, sorbic acid, and the like). Isotonic agents, for example, sugars, sodium chloride, and the like may also be included. Prolonged absorption of the injectable pharmaceutical form can be brought about by the use of agents delaying absorption (for example, aluminum monostearate and gelatin).

Solid dosage forms for oral administration include capsules, tablets, pills, powders and granules. In such solid dosage forms, the active compound is mixed with at least one inert excipient (or carrier) (for example, sodium citrate or dicalcium phosphate), or further mixed with (a) fillers or extenders (such as starch, lactose, sucrose, glucose, mannitol, and silicic acid); (b) binders (such as carboxymethyl cellulose, alginate, gelatin, polyvinylpyrrolidone, sucrose, and acacia); (c) humectants (such as glycerol); (d) disintegrating agents (such as agar-agar, calcium carbonate, potato or tapioca starch, alginic acid, certain synthetic silicates, sodium carbonate); (e) solutions retarding agents (such as paraffin); (f) absorption accelerators (such as quaternary ammonium compounds); (g) wetting agents (such as cetyl alcohol and glyceryl monostearate); (h) adsorbents (such as kaolin and bentonite) and (i) lubricants (such as talc, calcium stearate, magnesium stearate, solid polyethylene glycols, sodium lauryl sulfate) or mixtures thereof.

Solid compositions of a similar type may also be employed as fillers in soft-filled and hard-filled gelatin capsules using such as lactose as well as high molecular weight polyethylene glycols and the like.

Solid dosage forms (such as tablets, dragees, capsules, pills, and granules) can be prepared with coatings

and shells (such as enteric coatings and others known in the art). They may contain opacifying agents, and may also be such compositions that they release the active compound(s) in a certain part of the intestinal tract in a delayed manner. Examples of embedding compositions which can be used are polymeric substances and waxes. The active components can also be in micro-encapsulated form, if appropriate, with one or more of the above-mentioned excipients.

Liquid dosage forms for oral administration include pharmaceutically acceptable emulsions, solutions, dispersions, syrups and elixirs. In addition to the active compound, liquid dosage forms may contain inert diluents commonly used in the art (such as water or other solvents), solubilizers, and emulsifiers (such as ethanol, isopropanol, ethyl carbonate, ethyl acetate, benzyl alcohol, benzyl benzoate, propylene glycol, 1,3-butanediol, dimethylformamide), oils (in particular, cottonseed oil, groundnut oil, corn oil, olive oil, castor oil, and sesame oil), glycerol, tetrahydrofuran alcohol, polyethylene glycols, fatty acid esters of sorbitan, or mixtures thereof.

Besides such inert diluents, pharmaceutical dosage forms can also include, for example, wetting agents, emulsifying and suspending agents, aromatizer, flavoring and perfuming agents.

Suspensions, in addition to the active compounds, may contain suspending agents such as ethoxylated isostearyl alcohols, polyoxyethylene sorbitol, sorbitan esters, microcrystalline cellulose, aluminum metahydroxide, bentonite, agar-agar and tragacanth or a mixture of these substances, and the like.

Pharmaceutical dosage forms according to the present application may also include ointments, powders, sprays and inhalants. The active component is admixed under sterile conditions with a physiologically acceptable carrier and any preservatives, buffers or propellants as may be required.

The amount of active ingredients in pharmaceutical compositions and pharmaceutical dosage forms can be appropriately determined by those skilled in the art as needed, for example, each active ingredient is usually present in a pharmaceutical composition or dosage form in a therapeutically effective amount.

For example, the pharmaceutical composition according to the present application can be formulated into oral dosage forms (such as long-acting sustained-release oral preparations or oral dosage forms administrated 2 to 3 times per day), intravenous injection dosage forms, and intramuscular injection dosage forms.

In a preferred embodiment of the present application, the pharmaceutical composition according to the present application is a dosage form for oral administration. In a further preferred embodiment of the present application, the pharmaceutical composition according to the present application is an oral dosage form administrated once a day, an oral dosage form administrated 2 to 3 times a day, or a long-acting sustained-release oral preparation.

In a preferred embodiment of the present application, the pharmaceutical composition according to the present application is a dosage form for oral administration. In a further preferred embodiment of the present application, the pharmaceutical composition according to the present application is a dosage form for daily oral administration.

III. Uses of pharmaceutical compositions and dosage forms

It has been found that the pharmaceutical composition and dosage form according to the present application are useful for the treatment of chronic kidney disease (CKD), especially diabetic kidney disease (DN or DKD).

Thus, the second aspect of the present application relates to use of the combination of doxazosin (or a pharmaceutically acceptable salt thereof), pramipexole (or a pharmaceutically acceptable salt thereof) and metoprolol (or a pharmaceutically acceptable salt thereof) in the preparation of a pharmaceutical composition for treatment of chronic kidney disease, especially diabetic kidney disease, in a subject in need thereof.

The third aspect of the present application relates to use of the combination of doxazosin (or a pharmaceutically acceptable salt thereof), pramipexole (or a pharmaceutically acceptable salt thereof) and metoprolol (or a pharmaceutically acceptable salt thereof) in treatment of chronic kidney disease, especially diabetic kidney disease, in a subject in need thereof.

The fourth aspect of the present application relates to a method for treating chronic kidney disease, especially diabetic kidney disease, the method comprising administering a therapeutically effective amount of doxazosin (or a pharmaceutically acceptable salt thereof), pramipexole (or a pharmaceutically acceptable salt thereof) and metoprolol (or a pharmaceutically acceptable salt thereof) to a subject in need.

The pharmaceutical composition and dosage form according to the application are suitable for the treatment of diabetic kidney disease at various stages, and are also suitable for the preventive treatment before the onset of the disease.

A "patient" or "subject" treated by the methods according to the present application may refer to a human or a non-human animal, such as a primate. Preferably, the "patient" or "subject" is a human.

The treatment method according to the present application involves the combination of multiple active ingredients, also known as "combined therapy" or "combination therapy" or "co-therapy". The "combination medicine" or "combination therapy" refers to the administration of multiple active ingredients described in the present application so that they work together to provide beneficial effects. The beneficial effects of the above combination include, but are not limited to, pharmacokinetic or pharmacodynamic co-actions resulting from the combination of the above active ingredients. Administration of these therapeutic agents in combination typically is carried out over a defined time period (usually minutes, hours, days or weeks depending upon the physician's judgment). "Combination medicine" or "combination therapy" is intended to embrace administration of the active ingredients in a sequential manner, that is, wherein each active ingredient is administered at a different time; as well as administration of the active ingredients or at least two of these active ingredients, in a substantially simultaneous manner. Substantially simultaneous administration can be accomplished, for example, by administering to the subject a single capsule having a fixed ratio of each active ingredient, or in multiple, single capsules for each of the active ingredients. Sequential or substantially simultaneous administration of each active ingredient may be effected by any suitable route including, but not limited to, oral, intravenous, intramuscular, and direct absorption through mucous membrane tissues. The active ingredients may be administered by the same route or different routes. For example, a first active ingredient of a selected combination may be administered by intravenous injection, while the other active ingredients of the combination may be administered orally. Alternatively, for example, all active ingredients may be administrated orally, or all active ingredients may be administered by intravenous injection. The sequence in which these active ingredients are administrated is not strictly limited.

"Combined medicine" or "combination therapy" also includes the combined administration of the active ingredients as described above with other biologically active ingredients and non-drug therapies (such as surgery or mechanical treatments). Where the combination therapy further includes a non-drug treatment, the non-drug treatment may be carried out at any appropriate time, as long as a beneficial effect from the co-action of the combination of the active ingredients and non-drug treatment is achieved. For example, where appropriate, the beneficial effect is still achieved when the non-drug treatment is temporarily removed from the administration of the active ingredient, perhaps by days or even weeks.

In order to achieve the desired effect, it is usually necessary to administer a therapeutically effective amount of the pharmaceutical composition or dosage form according to the present application or each active ingredient alone to the patient or subject.

The phrase "therapeutically effective amount" is an art-recognized term. In certain embodiments, the term refers to an amount necessary or sufficient to eliminate, reduce or maintain a target of a particular treatment regime. The effective amount may vary depending on factors such as the disease or disorder being treated, the particular targeted constructs being administered, the size of the subject or the severity of the disease or disorder, and the like. The effective amount of a particular compound can be determined empirically by one of ordinary skill in the art or by a physician without necessitating undue experimentation. In certain embodiments, a therapeutically effective amount of a therapeutic agent for *in vivo* use will likely depend on a number of factors, including: the mode and method of administration; any other materials included in the medicament in addition to the agent. Optionally, *in vitro* or *in vivo* assays are used to help determine optimal dosage ranges.

In some embodiments of the present application, for an adult with a normal body weight of about 60 kg, when the pharmaceutical dosage form or pharmaceutical composition according to the present application is orally administered, the daily dose of doxazosin or a pharmaceutically acceptable salt thereof can range from 0.5 mg to 16 mg, for example can be 0.5 mg to 15 mg, 0.5 mg to 13 mg, 0.5 mg to 12 mg, 0.5 mg to 10 mg, 0.5 mg to 8 mg, 0.5 mg to 6 mg, 0.5 mg to 5 mg, 0.5 mg to 4 mg, 1 mg to 15 mg, 1 mg to 13 mg, 1 mg to 12 mg, 1 mg to 10 mg, 1 mg to 8 mg, 1 mg to 6 mg, 1 mg to 5 mg, 1 mg to 4 mg, 1.5 mg to 16 mg, 1.5 mg to 15 mg, 1.5 mg to 13 mg, 1.5 mg to 12 mg, 1.5 mg to 10 mg, 1.5 mg to 8 mg, 1.5 mg to 6 mg, 1.5 mg to 5 mg, 1.5 mg to 4 mg, 2 mg to 16 mg, 2 mg to 15 mg, 2 mg to 13 mg, 2 mg to 12 mg, 2 mg to 10 mg, 2 mg to 8 mg, 2 mg to 6 mg, 2 mg to 5 mg, 2 mg to 4 mg, etc. In a preferred embodiment of the present application, the daily dose of doxazosin or a pharmaceutically acceptable salt thereof is about 2 mg. In another preferred embodiment of the present application, the daily dose of doxazosin is about 4 mg.

In some embodiments of the present application, for an adult with a normal body weight of about 60 kg, when the pharmaceutical dosage form or pharmaceutical composition according to the present application is orally administered, the daily dose of pramipexole or a pharmaceutically acceptable salt thereof can range from 0.05 mg to 4.5 mg, for example can be 0.05 mg to 4.2 mg, 0.05 mg to 4 mg, 0.05 mg to 3.5 mg, 0.05 mg to 3 mg, 0.05 mg to 2.5 mg, 0.05 mg to 2 mg, 0.05 mg to 1.5 mg, 0.05 mg to 1 mg, 0.1 mg to 4.5 mg, 0.1 mg to 4.2 mg, 0.1 mg to 4 mg, 0.1 mg to 3.5 mg, 0.1 mg to 3 mg, 0.1 mg to 2.5 mg, 0.1 mg to 2 mg, 0.1 mg to 1.5 mg, 0.1 mg to 1 mg, 0.2 mg to 4.5 mg, 0.2 mg to 4.2 mg, 0.2 mg to 4 mg, 0.2 mg to 3.5 mg, 0.2 mg to 3 mg, 0.2 mg to 2.5 mg, 0.2 mg to 2 mg, 0.2 mg to 1.5 mg, 0.2 mg to 1.0 mg, 0.5 mg to 4.5 mg, 0.5 mg to 4.2 mg, 0.5 mg to 4 mg, 0.5 mg to 3.5 mg, 0.5 mg to 3 mg, 0.5 mg to 2.5 mg, 0.5 mg to 2 mg, 0.5 mg to 1.5 mg, 0.5 mg to 1 mg, 1 mg to 4 mg, 1 mg to 3 mg, 1 mg to 2.5 mg, etc. In a preferred embodiment of the present application, the daily dose of pramipexole or a pharmaceutically acceptable salt thereof is 0.1 mg to 1 mg. In a preferred embodiment of the present application, the daily dose of pramipexole is about 0.0625 mg. In another preferred embodiment of the present application, the daily dose of pramipexole is about 0.125 mg.

In some embodiments of the present application, for an adult with a normal body weight of about 60 kg, when the pharmaceutical dosage form or pharmaceutical composition according to the present application is orally administered, the daily dose of metoprolol or a pharmaceutically acceptable salt thereof can range from 5 mg to 200 mg, such as 5 mg to 180 mg, 5 mg to 160 mg,5 mg to 150 mg, 5 mg to 130 mg, 5 mg to 120 mg, 5 mg to 100 mg, 5 mg to 80 mg,

5 mg to 60 mg, 5 mg to 50 mg, 5 mg to 40 mg, 10 mg to 180 mg, 10 mg to 160 mg, 10 mg to 150 mg, 10 mg to 130 mg, 10 mg to 120 mg, 10 mg to 100 mg, 10 mg to 80 mg, 10 mg to 60 mg, 10 mg to 50 mg, 10 mg to 40 mg,15 mg to 200 mg, 15 mg to 180 mg, 15 mg to 160 mg, 15 mg to 150 mg, 15 mg to 130 mg, 15 mg to 120 mg, 15 mg to 100 mg, 15 mg to 80 mg, 15 mg to 60 mg, 20 mg to 200 mg, 20 mg to 180 mg,20 mg to 160 mg, 20 mg to 150 mg, 20 mg to 130 mg, 20 mg to 120 mg, 20 mg to 100 mg, 20 mg to 80 mg, 20 mg to 600 wt. In a preferred embodiment of the present application, the daily dose of metoprolol or a pharmaceutically acceptable salt thereof is 10 mg to 100 mg. In a preferred embodiment of the present application, the daily dose of metoprolol is about 20 mg. In another preferred embodiment of the present application, the daily dose of metoprolol is about 40 mg.

The above-mentioned daily dosage can be administered continuously periodically, for example once every 2 hours, every 6 hours, every 8 hours, every 12 hours, approximately every 24 hours. Preferably, the daily dose may be administered to the patient 2 to 3 times a day, or as a sustained-release tablet. The oral daily doses of the above three active ingredients are relatively different, which is determined by the pharmacokinetics of each active ingredient *in vivo.*

Those skilled in the art can understand that when the pharmaceutical composition according to the present application is formulated into other dosage forms such as intravenous infusion or intramuscular injection, the dosage range of each active ingredient may be different from the oral dosage range given above, and can be reasonably determined by a person skilled in the art or doctors combining *in vivo* and *in vitro* experimentation and taking into account the different pharmacokinetic characteristics of various routes of administration.

In a preferred embodiment of the present application, the pharmaceutical composition according to the present application is used for primate subjects, especially human subjects.

Those skilled in the art can understand that various aspects of the application described herein can be combined in various ways that are obvious to those skilled in the art without departing from the subject and idea of the application. Such combinations are also included within the scope of the present application. For example, the dosage ranges of certain components involved in this application include any combination of any lower limit and any upper limit mentioned in the specification, and also include arbitrary ranges constituted of particular amounts of the component mentioned in each specific example used as lower limits or upper limits. All these ranges obtained from such combinations fall within the scope of the invention. In addition, each feature of the present application listed in the specification can be combined with any other feature of the invention, and such combination is also within the disclosure scope of the present application.

## Examples

Rhesus monkeys with spontaneous chronic diabetic kidney disease were used as experimental animals to verify the effect of the combination of three marketed GPCR signaling pathway drugs including doxazosin (DOX), pramipexole (PMP) and metoprolol (MTP) on diabetic kidney disease and the drug safety and tolerance thereof. In the experiments, Valsartan, a commonly used first-line drug for DKD, was used as a positive control.

### I. Experimental materials

The following three marketed drugs were used as test articles in the experiments:

| | Drug Name | Action mode | Monkey dose (mg/kg) | Approximately equivalent human clinical dose (mg) |
|---|---|---|---|---|
| Test article 1 | Doxazosin (DOX) | α1-AR blocker | 0.133, 0.266 | 2 to 4 |
| Test article 2 | Pramipexole (PMP) | D3 receptor agonist | 0.002, 0.004 | 0.0625 to 0.125 |
| Test article 3 | Metoprolol (MTP) | β receptor blocker | 0.833, 1.677 | 10 to 50 |
| Note: The dosage for monkeys and humans can be converted by body surface area method or drug concentration exposure *in vivo.* | | | | |

Test drug 1:

Name or abbreviation: Doxazosin mesylate (DOX)
Purity: 98% HPLC
Manufacturer: Shanghai Ziqi Biotechnology Co., Ltd.

Test drug 2:

Name or abbreviation: Pramipexole hydrochloride (Pramipexole 2HCl Monohydrate, PMP)
Purity: 99.55% HPLC
Manufacturer: Shanghai Ziqi Biotechnology Co., Ltd.

Test drug 3:

Name or abbreviation: Metoprolol tartrate salt (MTP)
Purity: 98% HPLC
Manufacturer: Aladdin (Shanghai) Reagent Co., Ltd.
Positive control drug: Valsartan
Name or abbreviation: Valsartan
Manufacturer: Shanghai Bide Pharmatech Co., Ltd.

**II. Specific experiment protocol**

**Experimental animals:**

Animal species: Macaca mulatta (Rhesus Macaque).
Grade: conventional grade. The animals were quarantined before the test, including physical examination, 2 tubercle bacillus tests, parasites, salmonella, gillella and B virus inspection.
Animal identification: a stainless steel number plate with Arabic numerals engraved on the neck ring, and a tattoo on the chest were used.
Supplier: Ya'an Primed Biotechnology Co., Ltd.
Production license number: SCXK (Sichuan) 2019-027
The experiments complied with AAALAC requirements and GLP standards.

Environment:

Environmental grade: conventional grade. Temperature: 18 to 26°C. Relative humidity: 40% to 70%. Ventilation: the number of air changes per hour was not less than 8, using 100% fresh air (no air circulation). Lighting time: automatic lighting, alternating light and dark every 12 hours, with the light turned off at 7:30 PM and turned on at 7:30 AM of the next day. Animal cage: 850*900*2365 mm double-layer stainless steel cage. Stocking density: 1 monkey/cage.
A total of 16 rhesus monkeys with spontaneous chronic diabetic kidney disease (DKD) animals were enrolled, meeting the following inclusion criteria:

1) 16 males/females, aged 14 to 24 years (equivalent to adults aged 40 to 75); 14 males, weighing 7 to 13 kg; 2 females weighing 6 to 8 kg
2) With a diabetes duration of more than 2 years: fasting plasma glucose (FPG) of greater than 4.8 mmol/L, while age-matched controls with FPG 4.1 $\pm$ 0.3 mmol/L;
3) Grading G3a to G3b of CKD-EPI with glomerular filtration rate eGFR: 30 to 59 ml/min/1.73m$^2$, vs. age-matched controls with 92$\pm$11 ml/min/1.73m$^2$; or with moderate to severe increase in proteinuria (A2 to A3)with urinary albumin/creatinine ratio (UACR): 15 mg/g to 350 mg/g (4 to 6 hour urine collection), *vs.* age-matched controls 3 $\pm$ 2 mg/g.
4) With normal blood pressure, grade 1 or grade 2 hypertension (consistent with clinical patient standards).

**Exclusion criteria:**

1) Severe abnormal liver function
2) Any other diseases that may affect the efficacy evaluation

Table 1 below shows the basic etiology and characteristics of rhesus monkeys with spontaneous chronic diabetic kidney disease in each group at the baseline period.

Table 1 Baseline etiology and characteristics of rhesus monkeys with spontaneous chronic diabetic kidney disease in each group

| Group | Animal ID | Gender | Age (yrs) | Body Weigh t (kg) | CKD-EPI Grade # | UACR (mg/g) | eGFR (ml/min /1.73m$^2$) | CR-P (mg/dL) | CysC (mg/L) | Diabetic Duration (Years) | FPG (mmol/L) | Blood pressure (SBP/DBP) mmHg | RBC ($10^{12}$/L) | HGB (g/L) | ALB (g/L) | $K^+$ (mmol/L) |
|---|---|---|---|---|---|---|---|---|---|---|---|---|---|---|---|---|
| Experimental group 1# (n=4) | M1 | male | 21 | 9.40 | G3b-A3 | 303 | 34 | 1.25 | 2.70 | 5 | 5.13 | 172/85 | 5.99 | 132 | 39.0 | 5.70 |
| | M3 | male | 21 | 8.55 | G2-A2 | 116 | 86 | 0.59 | 1.09 | 7 | 12.50 | 146/68 | 5.26 | 128 | 37.6 | 4.26 |
| | M4 | male | 24 | 7.46 | G3a-A2 | 48 | 52 | 1.11 | 1.56 | 6 | 5.12 | 137/63 | 5.23 | 140 | 46.6 | 4.61 |
| | M2 | male | 22 | 7.93 | G3a-A2 | 15 | 57 | 0.93 | 1.63 | 7 | 4.88 | 111/63 | 5.61 | 133 | 41.5 | 4.58 |
| Experimental group 2# (n=4) | M5 | female | 20 | 7.06 | G3a-A2 | 203 | 50 | 0.98 | 1.54 | 3 | 5.54 | 141/58 | 5.73 | 150 | 43.4 | 5.60 |
| | M6 | female | 20 | 6.45 | G2-A2 | 44 | 74 | 0.74 | 1.11 | 6 | 9.64 | 153/74 | 4.88 | 122 | 44.2 | 4.23 |
| | M7 | male | 15 | 1126 | G3a | 3 | 58 | 1.17 | 1.54 | 4 | 4.81 | 119/58 | 6.05 | 143 | 46.4 | 4.31 |
| | M8 | male | 16 | 9.60 | G2-A2 | 39 | 83 | 0.87 | 1.14 | 6 | 5.67 | 163/77 | 6.22 | 168 | 45.9 | 5.38 |
| Valsartan group (n=4) | M9 | male | 17 | 10.38 | G3b-A2 | 80 | 43 | 1.79 | 1.55 | 4 | 5.31 | 149/71 | 5.45 | 127 | 49.3 | 4.72 |
| | M10 | male | 24 | 7.15 | G2-A2 | 98 | 66 | 0.67 | 1.45 | 2 | 5.13 | 147/91 | 6.33 | 147 | 38.9 | 5.15 |
| | M11 | male | 17 | 11.23 | G3a-A2 | 30 | 49 | 1.31 | 1.68 | 3 | 5.75 | 116/56 | 6.77 | 152 | 46.9 | 4.27 |
| | M12 | male | 18 | 9.35 | G3a-A2 | 37 | 57 | 1.16 | 1.55 | 4 | 7.77 | 129/67 | 6.79 | 153 | 42.8 | 4.63 |
| Placebo group (n=4) | M13 | male | 22 | 12.71 | G3a | 10 | 53 | 1.00 | 1.71 | 7 | 5.35 | 175/82 | 5.69 | 143 | 40.1 | 3.92 |
| | M14 | male | 21 | 11.88 | G3a-A2 | 28 | 47 | 1.42 | 1.55 | 2 | 4.86 | 143/65 | 6.09 | 140 | 46.2 | 4.38 |
| | M15 | male | 15 | 9.05 | G3a-A2 | 14 | 58 | 1.07 | 1.67 | 2 | 4.91 | 130/60 | 5.99 | 144 | 46.5 | 4.21 |
| | M16 | male | 14 | 12.58 | G3a-A2 | 16 | 57 | 1.15 | 1.64 | 4 | 4.86 | 139/57 | 5.98 | 143 | 46.2 | 4.38 |

**Notes: # G represents eGFR grade, A represents urine albumin grade, A2 represents moderate, and A3 represents severe.**

## Grouping and dosing regime:

In this trial, there were 3 groups including DOX+PMP+MTP group (n=8), Valsartan group (n=4) and placebo group (n=4). The DOX+PMP+MTP group was further divided into experimental group 1# and experimental group 2#. The details of the dosing regime of each group are described below, wherein the route of administration was oral administration. The baseline period was 1 month, followed by continuous oral administration for 58 days.

**Experimental group 1#: 4 animals**

D0 to D14: DOX+PMP+MTP: 0.133+0.002+0.833 mg/kg, twice a day;
D15 to D28: DOX+PMP+MTP: 0.266+0.002+1.667 mg/kg, twice a day;
D29 to D58: DOX+PMP+MTP: 0.266+0.004+0.833 mg/kg, DOX and MTP twice a day, PMP once a day

**Experimental group 2#: 4 animals**

D0 to D14: DOX+PMP+MTP: 0.133+0.002+0.833 mg/kg, twice a day;
D15 to D28: DOX+PMP+MTP: 0.133+0.002+1.667mg/kg, twice a day;
D29 to D58: DOX+PMP+MTP: 0.266+0.002+1.667mg/kg, twice a day;

**Valsartan group:** 4 animals were administered at the dose of 2.67 mg/kg (equivalent to the clinical equivalent dose of 40 to 80 mg), daily dose from D0 to D58, once a day in the first to second weeks of the administration period, and twice a day in the third to eighth weeks.

**Placebo group:** 4 animals were given with drinking water or fruit and observed continuously for 58 days.

In order to further evaluate the influence of each active ingredient in the pharmaceutical composition on drug efficacy, another group of rhesus monkeys with spontaneous chronic diabetic kidney disease was selected for the additional trial, in which the effects of administration of the DOX+MTP composition and placebo were compared in parallel.

The details of the dosing regime of each group are described below, wherein the route of administration was oral administration. The baseline period was 1 month, followed by continuous oral administration for 60 days.

**DOX+ MTP group:** 4 animals

D0 to D30: DOX+MTP: 0.266 mg/kg +1.667 mg/kg, twice a day;
D30 to D58: DOX+MTP: 0.532 mg/kg +3.334 mg/kg, twice a day;

**Placebo group:** 3 animals were given with drinking water or fruit and observed continuously for 60 days.

The baseline etiology and characteristics of each experimental animal in the additional trial were shown in Table 1A below.

**Table 1A Baseline etiology and characteristics of rhesus monkeys with spontaneous chronic diabetic kidney disease in each group of the additional trial**

| Group | Animal ID | Gender | Age (yrs) | Body Weight (kg) | CKD-EPI Grade # | UACR (mg/g) | eGFR (ml/min/ $1.73m^2$) | CRS (mg/dL) | CysC (mg/L) | Diabetic Duration (Years) | FPG (mmol/L) | Blood pressure (SBP/DBP) mmHg |
|---|---|---|---|---|---|---|---|---|---|---|---|---|
| DOX+MTP group (n=4) | M21 | female | 20 | 7.71 | G3-A2 | 84 | 50 | 0.85 | 1.74 | 3 | 5.25 | 138/57 |
| | M22 | male | 22 | 8.20 | G3-A2 | 207 | 33 | 1.03 | 3.23 | 5 | 5.15 | 158/67 |
| | M23 | male | 20 | 13.48 | G3-A2 | 170 | 49 | 1.24 | 1.67 | 4 | 5.25 | 170/80 |
| | M24 | female | 20 | 6.55 | G2-A2 | 73 | 65 | 0.70 | 1.39 | 3 | 5.91 | 135/67 |
| Placebo group (n=3) | M25 | female | 21 | 6.82 | G2-A2 | 17 | 76 | 0.71 | 1.08 | 3 | 4.86 | 143/65 |
| | M26 | female | 16 | 8.38 | G2-A3 | 334 | 66 | 0.78 | 1.33 | 3 | 4.96 | 160/70 |
| | M27 | female | 22 | 6.88 | G4-A2 | 72 | 24 | 1.55 | 3.07 | 4 | 5.35 | 175/82 |

**Notes: # G represents eGFR grade, A represents urine albumin grade, A2 represents moderate, and A3 represents severe.**

## III. Main observation indicators and examination methods

### 1. Main outcome measures

Glomerular filtration rate eGFR: creatinine (Cr-P), blood urea nitrogen (BUN), cystatin (CysC) were detected to calculate the eGFR value. Detections were performed once in the baseline period before administration, and at designated times (such as D14, D28, D45, and D58, etc.) after administration. In accordance with reference literature (Levey AS, Stevens LA, Schmid CH et al. A new equation to estimate glomerular filtration rate. Ann Intern Med 2009; 150:604-612), the formula for calculating eGFR was as follows:

$$eGFR_{male}=135 \times \min(Cr/0.9,1)^{-0.207} \times \max(Cr/0.9,1)^{-0.601} \times \min(CysC/0.8,1)^{-0.375} \times \max(CysC/0.8,1)^{-0.711} \times 0.995^{Age\times 3}$$

$$eGFR_{female}=135 \times \min(Cr/0.7,1)^{-0.284} \times \max(Cr/0.7,1)^{-0.601} \times \min(CysC/0.8,1)^{-0.375} \times \max(CysC/0.8,1)^{-0.711} \times 0.995^{Age\times 3} \times 0.969$$

Urinary albumin excretion rate (UACR): 4h to 6 hours urine was collected and detected for urinary microalbumin (Malb) and urinary creatinine (Cr-U), followed by calculation of UACR value. Detection was performed one time before administration, and at designated times (such as once at D30 and once at D58) after administration. UACR=Malb / Cr-U.

Blood pressure: blood pressure was detected after anesthesia, including systolic blood pressure (SBP), diastolic blood pressure (DBP), mean blood pressure (MBP) and heart rate (HR). Detection was carried out 1 time before administration and 1 time at D58 (end of administration).

### 2. Secondary outcome measures

Serum potassium: Detections were performed one time before administration, and at designated times (such as once at D14, D28, D45 and D58, respectively) after administration.

Glucose and lipid levels and liver function: FPG, FRA, LDL-c, HDL-c, TG, TC, ALT, AST, etc. Detections were performed one time before administration, and at designated times (such as once at D28 and once at D58) after administration.

Hematology: Detections were performed one time before administration, and at designated times (such as once at D28 and once at D58) after administration.

Body weight: one time before administration and once every 2 weeks during the administration period.

24-hour food intake and behavior were observed.

### 3. Sample Collection and Storage

Blood sample collection method: Before the blood collection operation, an animal was fasted overnight without anesthesia. After the animals were acclimatized, the backboard was gently squeezed to restrain the animal, and blood was collected through the forearm vein. After collection, sterilized dry cotton ball was used to gently press the blood collection site to stop bleeding.

Urine sample collection method: Animals were fasted overnight before the operation of collecting urine. On the day of operation, animals were transferred to a metabolic cage, followed by collecting all urine excreted within 4 to 6 hours into a urine collection bag.

Sample processing method: see Table 3-1

**Table 3-1 Sample collection and processing information**

| Sampling method | Sampling volume and temporary storage conditions | Type of collection tube | Sample processing method | Sample use |
|---|---|---|---|---|
| Fasting, without anesthesia | 2 ml ice water mix | EDTA2K anticoagulation Vacuum blood collection tube | Centrifuge at 3000 rpm for 10 min at 4°C to separate plasma | Renal function / Glycolipid / Liver function |

(continued)

| Sampling method | Sampling volume and temporary storage conditions | Type of collection tube | Sample processing method | Sample use |
|---|---|---|---|---|
| | 1 ml room temperature | EDTA2K anticoagulation Vacuum blood collection tube | / | Hematology |
| | 1 ml room temperature | Coagulation with separation gel Vacuum blood collection tube | Centrifuge at 5000 rpm for 10 min at 4°C to separate serum | Blood potassium |
| | 30 ml of urine, ice water mix | Centrifuge tube | 3000 rpm Instantaneously centrifuged | UACR related indicators |

**4. Detection method and equipment**

Detection method: see Tables 3-2 and 3-3

Hematology instrument: Siemens ADVIA 2120i Hematolagy Systems

Blood biochemistry and urinalysis instruments: Roche cobas6000 analyzer series C501, NT-proBNP was measured by ELISA.

**Table 3-2 Biochemistry items**

| Measurement items | Unit | Test methods: |
|---|---|---|
| Serum potassium ion (K+) | mmol/L | Indirect ion selective electrode method |
| Urine - Microalbumin (Malb) | g/L | Immunoturbidimetry, 2-point endpoint |
| Urine-creatinine (Cr-U) | mg/dL | Enzyme colorimetry |
| Blood urea nitrogen (BUN) | mg/dL | Colorimetry, rate method |
| Plasma creatinine (Cr-P) | mg/dL | Enzyme colorimetry |
| Cystatin C (CysC) | mg/L | Immunoturbidimetry |
| Total Cholesterol (TC) | mmol/L | Enzyme colorimetry |
| Triglycerides (TG) | mmol/L | Enzyme colorimetry |
| High-density lipoprotein (HDL-c) | mmol/L | Homogeneous enzyme colorimetric method |
| Low-density lipoprotein (LDL-c) | mmol/L | Homogeneous enzyme colorimetric method |
| Fasting plasma glucose (FPG) | mmol/L | Hexokinase method |
| Fructosamine (FRA) | μmol/L | Rate method |
| Alanine transaminase (ALT) | IU/L | IFCC rate method |
| Aspartate transaminase (AST) | IU/L | Colorimetry |
| Total Protein (TP) | g/L | Colorimetry, 2-point endpoint |
| Albumin (ALB) | g/L | Colorimetry |

**Table 3-3 Hematology items**

| Indicators | Unit | Test methods: |
|---|---|---|
| Red blood cell count (RBC) | 10^12/L | 2D laser |
| Hemoglobin (HGB) | g/L | Cyanmethemoglobin |

**5. Blood pressure**

Detection method: Animals were anesthetized by intramuscular injection of 15 mg/kg ketamine hydrochloride and then placed in a supine position, followed by being tied with a cuff of appropriate size according to the corresponding

standard. A blood oxygen probe was clamped on a finger or toe (except the left hand) of the animal, with the red photosensitive surface on the side of the finger pad. The blood pressure of the animal was continuously detected 3 times in automatic mode with an interval of 1 minute. If the differences in the SBP, DBP and MBP of the three times were less than 15 mmHg, the measurement completed, otherwise the measurement repeated.

Detection indicators: systolic blood pressure (SBP), diastolic blood pressure (DBP), mean blood pressure (MBP) and heart rate (HR).

Instrument: GE B40i electrophysiological monitor.

### 6. Clinical observation

Observation times: once a day.

Observation method: cage-side observation.

Observation indicators: injection site, skin, clothing hair, eyes, ears, nose, mouth, chest, abdomen, urogenital, limbs and other parts, as well as breathing, exercise, urination, defecation and behavior changes.

### 7. Body weight

Weighing time: before animals were fed on the day of weighing.

Measuring method: animals were fasted for 14 to 16 hours before weighing, and animals were weighed with a large animal scale after entering the transfer cage in an awake state.

Measuring instrument: METTLER TOLEDO electronic scale.

### 8. Data processing

Experimental results were presented individually. All measurement data were represented by "Mean ± SD" (mean ± standard deviation), and paired t-tests were performed to assess differences between before and after administration. $P < 0.05$ was considered as statistically significant.

## IV. Results and discussion

### 1. Effects on proteinuria (UACR)

Proteins in urine will increase the osmotic pressure of urine, affecting the concentration function of the kidney, and causing damage to the function of glomeruli and renal tubules. Proteinuria is an independent risk factor for glomerular fibrosis. Therefore, controlling UACR is a very important therapeutic strategy for reducing the risk of renal fibrosis and delaying the progression of end-stage renal failure.

Effects on urinary albumin excretion rate (UACR) are shown in Fig. 1, Fig. 2 and Table 4-1. DKD rhesus monkeys with moderate to severe increase in proteinuria, manifested as baseline urine albumin/creatinine ratio (UACR) in the range of 15 mg/g to 350 mg/g (in 4 to 6 hours urine collection) in each group were selected and included to analyze UACR changes at D30 and D58 from baseline. The results are as follows:

Placebo group (n=4): Compared with the baseline, the UACR (mg/g) at D58 increased by an average of 27.16 ± 23.57%; the UACR level of 4 animals fluctuated within a certain range and did not show rapid progress.

Valsartan group (n=4, and 3 in 4 monkeys with moderate to severe increase in proteinuria included for statistical analysis): Compared with the baseline, the UACR decreased by an average of about 30% at D30 and the UACR at D58 decreased by an average of 40.71 ± 8.27%, which was significantly lower than that of the placebo group ($p<0.01$).

DOX+PMP+MTP experimental group 1# (n=4, 4/4 monkeys with moderate to severe increase in proteinuria included for statistical analysis): Compared with the baseline, the UACR decreased by an average of 50.57 ± 23.86% at D30 after administration, which was significantly lower than that of the placebo group ($p<0.01$), and remained significantly lower, until the UACR at D58 decreased by an average of 68.75 ± 12.73%, which was significantly lower than that of the placebo group ($p<0.01$).

DOX+PMP+MTP experimental group 2# (n=4, 3/4 monkeys with moderate to severe increase in proteinuria included for statistical analysis): Compared with the baseline, the UACR decreased by an average of 41.56 ± 17.57% at D30 after administration, which was significantly lower than that of the placebo group ($p<0.01$), and remained significantly lower, until the UACR at D58 decreased by an average of 42.37 ± 3.50%, which was significantly lower than that of the placebo group ($p<0.01$).

In conclusion, administration of DOX+PMP+MTP for 30 days can significantly improve proteinuria, and the curative effect can last at least 58 days after administration.

In addition, UACR results of the additional experiment (DOX+MTP group vs placebo group) are summarized

in Table 4-1A.

Placebo group (n=3): The UACR (mg/g) at D58 increased by an average of 44.26 ± 52.39 from baseline.

DOX+MTP group (n=4): The UACR at D30 after administration increased by an average of 27.10±75.86 (mg/g) from baseline, showing no statistical difference compared with the placebo group; The UACR at D58 after administration increased by an average of 11.44 ± 38.86 (mg/g) from baseline, showing no statistical difference compared with the placebo group.

DOX+MTP administration for 58 days did not show a significant effect on improving proteinuria in rhesus monkeys with diabetic kidney disease.

**Table 4-1 Effects of DOX+PMP+MTP administration for 58 days on proteinuria (UACR) in rhesus monkeys with spontaneous chronic diabetic kidney disease**

| Group | Animal ID | UACR (mg/g) | | | UACR change rate (%) | | Cr-U (mg/dL) | | | Malb (mg/L) | | |
|---|---|---|---|---|---|---|---|---|---|---|---|---|
| | | Baseline | D30 | D58 | Baseline-D30 | Baseline-D58 | Baseline | D30 | D58 | Baseline | D30 | D58 |
| DOX+PMP+MTP Experimental group 1# (n=4) | M1 | 303 | 182 | 144 | -39.90 | -52.28 | 47.1 | 152.2 | 115.9 | 142.5 | 276.8 | 167.3 |
| | M3 | 116 | 16 | 26 | -85.80 | -77.77 | 151.9 | 109.9 | 13.2 | 176.1 | 18.1 | 3.4 |
| | M4 | 48 | 27 | 10 | -43.37 | -79.75 | 19.7 | 26.3 | 84.0 | 9.4 | 7.1 | 8.1 |
| | M2 | 15 | 10 | 5 | -33.21 | -65.20 | 73.7 | 86.3 | 130.0 | 11.4 | 8.9 | 7.0 |
| | Mean±SD | 120±128 | 59±82 | 46±66 | -50.57±23.86 | -68.75±12.73 | 73.1±57.0 | 93.7±52.5 | 85.8±52.1 | 84.9±87.1 | 77.7±132.8 | 46.5±80.6 |
| | p-value | / | **0.1204** [a] | **0.1074** [a] | **0.0025** [b] | **0.0004** [b] | / | / | / | / | / | / |
| DOX+PMP+MTP Experimental group 2# (n=4) | M6 | 44 | 17 | 25 | -61.68 | -43.52 | 23.5 | 45.1 | 216.1 | 10.2 | 7.6 | 53.7 |
| | M8 | 39 | 26 | 24 | -33.75 | -38.44 | 30.9 | 142.4 | 86.2 | 12.1 | 36.8 | 20.7 |
| | M7* | 3 | 4 | 4 | NA | NA | 39.5 | 40.3 | 80.2 | 1.3 | 1.8 | 3.4 |
| | M5 | 203 | 144 | 111 | -29.25 | -45.15 | 16.1 | 84.3 | 19.2 | 32.7 | 121.1 | 21.4 |
| | Mean±SD | 72±89 | 48±65 | 41±48 | -41.56±17.57 | -42.37±3.50 | 27.5±10.0 | 78.0±47.2 | 100.4±82.8 | 14.1±13.3 | 41.8±55.0 | 24.8±21.0 |
| | p-value | / | **0.5909** [a] | **0.1359** [a] | **0.0085** [b] | **0.0116** [b] | / | / | / | / | / | / |
| Valsartan group (n=4) | M9 | 80 | 59 | 38 | -27.11 | -52.26 | 44.8 | 87.5 | 143.0 | 36.0 | 51.3 | 54.9 |
| | M10 | 98 | 84 | 64 | -14.25 | -34.73 | 252.5 | 238.1 | 323.6 | 247.1 | 199.8 | 206.7 |
| | M11 | 30 | 20 | 19 | -33.78 | -34.73 | 47.2 | 68.3 | 206.3 | 14.0 | 13.4 | 39.9 |
| | M12 | 37 | 21 | 22 | -42.53 | -41.10 | 19.0 | 27.5 | 28.2 | 7.1 | 5.9 | 6.2 |
| | Mean±SD | 61±33 | 46±31 | 36±20 | -29.41±11.92 | -40.71±8.27 | 90.9±108.5 | 105.3±92.0 | 175.3±123.4 | 76.1±114.7 | 67.6±90.3 | 76.9±88.9 |
| | p-value | / | **0.0082** [a] | **0.0432** [a] | **0.0009** [b] | **0.0016** [b] | / | / | / | / | / | / |
| Placebo group (n=4) | M13 | 10 | 12 | 15 | 17.87 | 45.48 | 42.0 | 41.3 | 40.7 | 4.4 | 5.1 | 6.2 |
| | M14 | 28 | 30 | 38 | 6.59 | 34.45 | 207.8 | 152.3 | 24.6 | 59.0 | 46.1 | 9.4 |
| | M15 | 14 | 16 | 20 | 7.18 | 36.17 | 49.7 | 54.1 | 51.2 | 7.2 | 8.4 | 10.1 |
| | M16 | 16 | 18 | 15 | 8.87 | -7.44 | 41.5 | 39.8 | 38.9 | 6.8 | 7.1 | 5.9 |
| | Mean±SD | 17±8 | 19±8 | 22±11 | 10.13±5.25 | 27.16±23.57 | 85.2±81.8 | 71.9±54.0 | 38.9±10.9 | 19.4±26.5 | 16.7±19.7 | 7.9±2.2 |
| | p-value | / | / | / | / | / | / | / | / | / | / | / |

Notes: 1. UACR=Cr-U/Malb; 2 "a" compared with baseline; 3 "b" compared with placebo group. 4. "*", The baseline UACR of animal in the experimental group 2# was < 10mg/kg and the results were not included in the statistical analysis of average and significant difference. 5. NA, UACR did not meet the inclusion criteria, thus was not included in the statistical analysis. 6. Change rate = (value at the time point after administration - baseline value) / baseline value * 100%, wherein a negative value indicating a decrease; 7. "#" represents $p<0.05$ compared with the placebo group.

**Table 4-1A DOX+MTP administration for 58 days on proteinuria (UACR) in rhesus monkeys with spontaneous chronic diabetic kidney disease**

| Group | Animal ID | UACR (mg/g) | | | UACR change rate (%) | | Cr-U (mg/dL) | | | Malb (mg/L) | | |
|---|---|---|---|---|---|---|---|---|---|---|---|---|
| | | Baseline | D30 | D58 | Baseline-D30 | Baseline-D58 | Baseline | D30 | D58 | Baseline | D30 | D58 |
| DOX+MTP Experimental group (n=4) | M21 | 84 | 190 | 138 | 126.19 | 64.29 | 152.5 | 132.5 | 132.2 | 182.2 | 80.3 | 95.8 |
| | M22 | 207 | 297 | 240 | 43.48 | 15.94 | 108.2 | 136.2 | 126.8 | 52.3 | 36.4 | 52.8 |
| | M23 | 170 | 145 | 151 | -14.71 | -11.18 | 60.5 | 67.5 | 53.7 | 35.5 | 41.7 | 35.6 |
| | M24 | 73 | 39 | 56 | -46.58 | -23.29 | 79.1 | 51.1 | 43.1 | 108.6 | 202.8 | 77.0 |
| | Mean±SD | 133±66 | 168±107 | 146±75 | 27.10±75.86 | 11.44±38.86 | 100.1±40.1 | 96.8±43.9 | 88.9±47.1 | 94.6±66.2 | 90.3±77.5 | 65.3±26.5 |
| | p-value | / | **0.4234** [a] | **0.5363** [a] | **0.9829** [b] | **0.3841** [b] | / | / | / | / | / | / |
| Placebo group (n=3) | M25 | 17 | 27 | 28 | 58.82 | 64.71 | 59.2 | 78 | 64.0 | 348.2 | 288.9 | 228.5 |
| | M26 | 334 | 301 | 283 | -9.88 | -15.27 | 195.1 | 201.9 | 176.9 | 58.4 | 67.1 | 62.5 |
| | M27 | 72 | 98 | 132 | 36.11 | 83.33 | 65.2 | 76.1 | 79.9 | 90.6 | 77.7 | 60.5 |
| | Mean±SD | 141±169 | 142±142 | 148±128 | 28.35±35.00 | 44.26±52.39 | 106.5±76.8 | 118.7±72.1 | 106.9±61.1 | 165.7±158. | 144.5±125. | 117.2±96.4 |
| | p-value | / | / | / | / | / | / | / | / | / | / | / |

Notes: 1. UACR=Cr-U/Malb; 2. "a" compared with baseline; 3. "b" compared with placebo group. 4. Change rate = (value at the given time point after administration - baseline value) / baseline value * 100%, wherein a negative value indicating a decrease.

**2. Effect on glomerular filtration rate eGFR**

Results of eGFR are shown in Fig. 3, Table 4-2 and Table 4-3. eGFR values of 30 to 59 ml/min/1.73m$^2$ were selected and included to analyze the changes of glomerular filtration rate.

Placebo group (n=4): Compared with the baseline, the glomerular filtration rate (eGFR) fluctuated within a certain range in the 4 animals and did not show rapid progress.

Valsartan group (n=4, 3 in 4 animals with eGFR of 30 to 59 ml/min/1.73m$^2$, included for statistical analysis): The glomerular filtration rate eGFR at D58 after administration increased by an average of 10 ± 7 ml/min/1.73 m$^2$ from baseline, which was significantly higher than that of the placebo group ($p<0.05$). Glomerular deterioration was significantly improved.

DOX+PMP+MTP experimental group 1# and experimental group 2# (eGFR of 30 to 59 ml/min/1.73m$^2$ were selected and included; and the DOX+PMP+MTP experimental group 1# and the DOX+PMP+MTP experimental group 2# were combined as one group for analysis with n=5): The glomerular filtration rate eGFR at D58 after administration increased by an average of 10 ± 4 ml/min/1.73 m$^2$ from baseline, which was significantly higher than that of the placebo group ($p<0.05$).

In conclusion, DOX+PMP+MTP administration for 58 days can significantly improve the glomerular filtration rate eGFR.

**Table 4-2 Effects of DOX+PMP+MTP administration for 58 days on eGFR in rhesus monkeys with spontaneous chronic diabetic kidney disease**

| Group | | eGFR (ml/min/1.73m$^2$) | | | | |
|---|---|---|---|---|---|---|
| | | **Baseline** | **D14** | **D28** | **D45** | **D58** |
| DOX+PMP+MTP Experimental group 1# (n=4) | Mean±SD | 48±12 | 57±15 | 55±14 | 56±15 | 60±14 |
| | p-value [a] | / | **0.0290** | **0.0459** | **0.0823** | **0.0176** |
| DOX+ PMP+MTP Experimental group 2# (n=4) | Mean±SD | 50±10 | 58±11 | 56±10 | 57±11 | 60±10 |
| | p-value [a] | / | **0.0753** | **0.1232** | **0.1606** | **0.1779** |
| Valsartan group (n=4) | Mean±SD | 50±7 | 53±7 | 56±2 | 55±1 | 60±1 |
| | p-value [a] | / | **0.1164** | **0.2411** | **0.2686** | **0.1253** |
| Placebo group (n=4) | Mean±SD | 54±5 | 57±6 | 53±7 | 55±5 | 54±7 |
| | p-value [a] | / | **0.0408** | **0.7951** | **0.1105** | **0.5571** |
| Notes: 1. "a" compared with baseline. | | | | | | |

**Table 4-3 eGFR change values of DOX+PMP+MTP administration for 58 days in rhesus monkeys with spontaneous chronic diabetic kidney disease**

| Group | | eGFR change value (ml/min/1.73m$^2$) | | | | |
|---|---|---|---|---|---|---|
| | | **Baseline** | **D14** | **D28** | **D45** | **D58** |
| DOX+ PMP+MTP group (n=5) | Mean±SD | 0±0 | 8±3 | 6±3 | 7±4 | 10±4 |
| | p-value [a] | / | **0.0393** | **0.0154** | **0.0261** | **0.0075** |
| Valsartan group (n=3) | Mean±SD | 0±0 | 3±2 | 6±7 | 5±6 | 10±7 |
| | p-value [a] | / | **0.9721** | **0.1229** | **0.2626** | **0.0435** |
| Placebo group (n=4) | Mean±SD | 0±0 | 3±2 | 0±3 | 1±1 | 1±2 |
| Notes: Animals with eGFR between 30 and 59 ml/min/1.73m$^2$ in DOX+PMP+MTP group wereincluded for statistical analysis. 1. "a" compared with baseline. 2. Change value = value after administration - baseline value | | | | | | |

In addition, eGFR results of the additional experiment (DOX+MTP group vs placebo group) are summarized in Table 4-2A. DOX+MTP administration for 30 and 58 days did not show a significant effect on improving eGFR in rhesus monkeys with diabetic kidney disease.

**Table 4-2A Effects of DOX+MTP administration for 58 days on eGFR in rhesus monkeys with spontaneous chronic diabetic kidney disease**

| group | | eGFR (ml/min/1.73m$^2$) | | |
|---|---|---|---|---|
| | | Baseline | D30 | D58 |
| DOX + MTP Experimental group (n=4) | Mean±SD | 49±13 | 49±15 | 50±13 |
| | p-value [a] | / | **0.8006** | **0.2522** |
| Placebo group (n=3) | Mean±SD | 55±28 | 57±27 | 57±27 |
| | p-value [a] | / | **0.2254** | **0.5876** |
| Note: 1. "a" compared with baseline. | | | | |

**3. Effects on CysC, Cr-P and BUN**

Effects on CysC, Cr-P and BUN are shown in Table 4-4 and Table 4-6. CysC is an endogenous marker that reflects changes in glomerular filtration rate. Cr-P has been used as the main index of renal function for more than 40 years. In this test, glomerular filtration rate eGFR is calculate by measuring CysC and Cr-P. Although BUN was first used as an evaluation index of renal function, it could not meet the requirements of endogenous GFR markers and was only used as an auxiliary index in this test.

**Table 4-4 Effects of administration for 58 days on CysC in rhesus monkeys with spontaneous chronic diabetic kidney disease**

| Group | | CysC (mg/L) | | | | |
|---|---|---|---|---|---|---|
| | | Baseline | D14 | D28 | D45 | D58 |
| DOX+ PMP+MTP Experimental group 1# (n=4) | Mean±SD | 1.96±0.64 | 1.76±0.69 | 1.83±0.68 | 1.77±0.66 | 1.63±0.56 |
| | p-value [a] | / | **0.0185** | **0.0588** | **0.0536** | **0.0165** |
| DOX+ PMP+MTP Experimental group 2# (n=4) | Mean±SD | 1.79±0.51 | 1.64±0.51 | 1.72±0.51 | 1.66±0.49 | 1.58±0.40 |
| | p-value [a] | / | **0.2048** | **0.8743** | **0.5000** | **0.6051** |
| Valsartan group (n=4) | Mean±SD | 1.57±0.10 | 1.59±0.10 | 1.53±0.08 | 1.56±0.09 | 1.48±0.11 |
| | p-value [a] | / | **0.7618** | **0.6013** | **0.8766** | **0.1181** |
| Placebo group (n=4) | Mean±SD | 1.64±0.07 | 1.63±0.04 | 1.65±0.03 | 1.63±0.14 | 1.67±0.09 |
| | p-value [a] | / | **0.5657** | **0.8437** | **0.7615** | **0.6619** |
| Note: 1. "a" compared with baseline. | | | | | | |

**Table 4-5 Effects of administration for 58 days on CR-P in rhesus monkeys with spontaneous chronic diabetic kidney disease**

| Group | CR-P (mg/dL) | | | | |
|---|---|---|---|---|---|
| | Baseline | D14 | D28 | D45 | D58 |
| DOX+ PMP+MTP Experimental group 1# (n=4) | 1.10±0.16 | 0.90±0.16 | 0.93±0.14 | 0.92±0.17 | 0.90±0.10 |
| DOX+ PMP+MTP Experimental group 2# (n=4) | 1.09±0.13 | 0.93±0.14 | 0.94±0.14 | 0.94±0.17 | 0.92±0.13 |
| Valsartan group (n=4) | 1.46±0.29 | 1.26±0.24 | 1.18±0.13 | 1.18±0.12 | 1.10±0.08 |
| Placebo group (n=4) | 1.16±0.18 | 1.07±0.19 | 1.18±0.22 | 1.13±0.17 | 1.13±0.21 |

**Table 4-6 Effects of administration for 58 days on BUN in rhesus monkeys with spontaneous chronic diabetic kidney disease**

| Group | BUN (mg/dL) | | | | |
|---|---|---|---|---|---|
| | Baseline | D14 | D28 | D45 | D58 |
| DOX+ PMP+MTP Experimental group 1# (n=4) | 46.0±17.0 | 38.8±19.0 | 37.2±16.4 | 33.5+11.3 | 31.3±10.4 |
| DOX+ PMP+MTP Experimental group 2# (n=4) | 27.6±4.8 | 23.7±3.8 | 26.3±3.9 | 23.4±2.0 | 24.6±2.0 |
| Valsartan group (n=4) | 41.9±34.2 | 27.1±6.0 | 35.0±16.6 | 37.9±19.2 | 31.6±11.3 |
| Placebo group (n=4) | 34.9±5.0 | 29.2±8.6 | 36.9±2.6 | 28.6±8.6 | 35.6±5.1 |

**4. Effects on blood pressure**

Effects on blood pressure are shown in Table 4-7 and Table 4-8.

Placebo group (n= 4, 2 in 4 monkeys with hypertension): Compared with the baseline, the blood pressure of 4 animals fluctuated within a certain range in the 4 animals.

Valsartan group (n=4, 2 in 4 monkeys with hypertension): Compared with the baseline, the SBP decreased by an average of 13 ± 6 mmHg at D58 after administration, which was significantly lower than that of the placebo group ($p<0.05$); DBP decreased by an average of 8 ± 6 mmHg, which was significantly lower than that of the placebo group ($p<0.01$).

DOX+PMP+MTP experimental group 1# (n=4, 2 in 4 monkeys with hypertension): Compared with the baseline, the SBP at D58 decreased by an average of 10 ± 3 mmHg, and the decrease was significantly lower than that of the placebo group ($P<0.05$ ); DBP did not exhibit a decrease. There was no risk of hypotension in normotensive animals.

DOX+PMP+MTP experimental group 2# (n=4, 2 in 4 monkeys with hypertension): Compared with the baseline, the SBP at D58 decreased by an average of 5 ± 5 mmHg, and the decrease was not statistically significant compared with that of the placebo group. DBP did not exhibit a decrease. The activity of decreasing blood pressure was weak.

In conclusion, the DOX+PMP+MTP composition had a certain activity of decreasing high pressure by 5 to 10 mmHg.

**Table 4-7 Grades of hypertension in rhesus monkeys**

| Classification | SBP (mmHg) | | DBP (mmHg) |
|---|---|---|---|
| normal blood pressure | < 120 | and | < 60 |
| high normal blood pressure | 120 to 139 | and/or | 60 to 69 |
| Grade 1 hypertension | 140 to 159 | and/or | 70 to 79 |
| Grade 2 hypertension | 160 to 179 | and/or | 80 to 89 |
| Grade 3 hypertension | ≥ 180 | and/or | ≥ 90 |
| isolated systolic hypertension | ≥ 140 | and | < 70 |
| Note: When SBP and DBP belong to different grades, the higher grade shall prevail. | | | |

**Table 4-8 Effects of administration for 58 days on blood pressure in rhesus monkeys with spontaneous chronic diabetic kidney disease**

| Group | Animal ID | SBP (mmHg) | | | DBP (mmHg) | | |
|---|---|---|---|---|---|---|---|
| | | Baseline | D58 | change value | Baseline | D58 | change value |
| DOX+ PMP+MTP Experimental group 1# (n=4) | Mean±SD | 142±25 | 132±22 | -10±3 | 70±10 | 68±8 | -2±3 |
| | p-value [a] | / | / | **0.0395** | / | / | **0.1024** |

(continued)

| Group | Animal ID | SBP (mmHg) | | | DBP (mmHg) | | |
|---|---|---|---|---|---|---|---|
| | | Baseline | D58 | change value | Baseline | D58 | change value |
| DOX+ PMP+MTP Experimental group 2# (n=4) | Mean±SD | 144±19 | 140±23 | -5±5 | 67±10 | 70±12 | 3±4 |
| | p-value [a] | / | / | **0.3667** | / | / | **1.0000** |
| Valsartan group (n=4) | Mean±SD | 135±16 | 123±12 | -13±6 | 71±15 | 63±11 | -8±5 |
| | p-value [a] | / | / | **0.0453** | / | / | **0.0076** |
| Placebo group | Mean±SD | 147±20 | 147±16 (n=4) | 0±8 | 66±11 | 69±13 | 3±3 |
| Note: "a" compared with the placebo group. | | | | | | | |

**5. Effects on $K^+$**

The effects on $K^+$ is shown in Table 4-9. Compared with the baseline period, there was no significant change in the serum $K^+$ levels of animals in each experimental group at D58 after administration. In conclusion, DOX+PMP+MTP administration for 58 days did not cause the risk of hyperkalemia.

**Table 4-9 Effects of administration for 58 days on $K^+$ in rhesus monkeys with spontaneous chronic diabetic kidney disease**

| Group | K ion (mg/dL) | | | | |
|---|---|---|---|---|---|
| | Baseline | D14 | D28 | D45 | D58 |
| DOX+ PMP+MTP Experimental group 1# (n=4) | 4.79±0.63 | 4.76 ± 0.54 | 4.64±0.60 | 4.45±0.48 | 4.54±0.12 |
| DOX+ PMP+MTP Experimental group 2# (n=4) | 4 88±0.71 | 4.94±0.80 | 4.87±0.34 | 4.70±0.15 | 4.74±0.37 |
| Valsartan group (n=4) | 4.69±0.36 | 4.70±0.79 | 4.62±0.89 | 4.97±1.08 | 5.00± 1.40 |
| Placebo group (n=4) | 4.22±0.22 | 4.02±0.44 | 4.11±0.59 | 4.16±0.27 | 4.11±0.51 |

**6. Safety and tolerance study**

The safe dosage ranges as well as toxic and side effects of DOX, PMP, and MTP as marketed drugs are known. The commonly used dose of DOX is 4 to 8 mg/time, 2 times a day. The recommended individual dose of PMP should be 0.375 mg to 4.5 mg per day. For the treatment of idiopathic Parkinson's disease, the initial dose is 0.375 mg per day, and then the dose is increased every 5 to 7 days, with a maximum dose of 1.5 mg. The incidence of somnolence increases at a daily dose of above 1.5 mg. The recommended dose of MTP is 50 to 200 mg/day, and can reach 300 mg/day or 400 mg/day if necessary, with known adverse reaction being hypotension.

In this experiment, the dosage of DOX, PMP and MTP combination to treat rhesus monkeys with chronic diabetic kidney disease, when calculated as the equivalent human dose for clinical patients, was far lower than the maximum value of the above-mentioned recommended dose, so theoretically the three-drug combination of DOX+PMP+MTP is very safe.

In addition, no drug-related adverse event was observed during the whole administration period, and no obvious change in liver function, kidney function, body weight, biochemical indicators (including FPG, LDL, HDL, TC, TG, ALT, AST, TP, ALB, etc.) was seen, further confirming the indeed good biological safety for the three-drug combination of DOX+PMP+MTP according to the present application.

## V. Conclusion

The above experimental results demonstrate that oral administration of three GPCR agonists (DOX+PMP+MTP) for 58 days to treat rhesus monkeys with spontaneous diabetic kidney disease can significantly reduce moderate to severe proteinuria and significantly improve the deterioration of glomerular filtration rate, inhibit the occurrence of end-stage renal failure; while, at the same time, it was safe and well-tolerated, and no hyperkalemia and fluid retention were seen during the treatment.

## VI. Pharmaceutical Composition Example 1

For a 10kg monkey, 1.5 mg of doxazosin mesylate, 0.02 mg of pramipexole dihydrochloride API powder, and 8 mg of metoprolol tartrate were uniformly mixed for Pharmaceutical Composition Example 1. The pharmaceutical composition can be directly mixed into food for administration, or can be prepared into oral tablets after being mixed with appropriate excipients or additives.

Although the specific embodiments have been described in detail above, professionals would understand that based on the disclosure and guide of the foregoing specification, professionalswould also make appropriate changes and modifications to the foregoing embodiments. Therefore, the present application is not limited to the specific embodiments disclosed and described above, and some modifications and changes to the present application also fall within the protection scope of the claims of the present application.

## Claims

1. A pharmaceutical composition for treating chronic kidney disease, comprising doxazosin or a pharmaceutically acceptable salt thereof, pramipexole or a pharmaceutically acceptable salt thereof, and metoprolol or a pharmaceutically acceptable salt thereof as active ingredients.

2. The pharmaceutical composition according to claim 1, comprising 0.5 to 45 parts by weight (preferably 1 to 10 parts by weight) of pramipexole or a pharmaceutically acceptable salt thereof, 5 to 160 parts by weight (preferably 5 parts by weight to 80 parts by weight) of doxazosin or a pharmaceutically acceptable salt thereof, and 50 parts by weight to 2000 parts by weight (preferably 100 parts by weight to 1000 parts by weight) of metoprolol or a pharmaceutically acceptable salt thereof.

3. The pharmaceutical composition according to claim 1 or 2, which is prepared as an oral dosage form or intravenous injection, preferably an oral dosage form.

4. The pharmaceutical composition according to any one of claims 1 to 3, wherein the pharmaceutically acceptable salt is selected from a group consisting of hydrochloride, sulfate, phosphate, pyrophosphate, hydrobromate or nitrate, citrate, fumarate, maleate, malate, ascorbate, succinate, tartrate, benzoate, acetate, methanesulfonate, ethanesulfonate, salicylate, stearate, benzenesulfonate or p-toluenesulfonate.

5. The pharmaceutical composition according to any one of claims 1 to 4, wherein the chronic kidney disease is diabetic kidney disease.

6. Use of a combination of doxazosin or a pharmaceutically acceptable salt thereof, pramipexole or a pharmaceutically acceptable salt thereof and metoprolol or a pharmaceutically acceptable salt thereof in the preparation of a pharmaceutical composition for treatment of chronic kidney disease in a subject in need thereof.

7. The use according to claim 5, wherein said pharmaceutical composition is an oral dosage form or intravenous injection dosage form, preferably an oral dosage form.

8. The use according to any one of claims 6 to 7, wherein the pharmaceutical composition is suitable for primate subjects, especially human subjects.

9. The use according to claim 8, wherein a daily dose of doxazosin or a pharmaceutically acceptable salt thereof is in a range from 0.5 mg to 16 mg, preferably from 0.5 mg to 8 mg, a daily dose of pramipexole or a pharmaceutically acceptable salt thereof is in a range from 0.05 mg to 4.5 mg, preferably from 0.1 mg to 1 mg, and a daily dose of metoprolol or a pharmaceutically acceptable salt thereof is in a range from 5 mg to 200 mg, preferably from 10 mg

to 100 mg.

**10.** The use according to any one of claims 6 to 9, wherein the pharmaceutically acceptable salt is selected from a group consisting of hydrochloride, sulfate, phosphate, pyrophosphate, hydrobromate or nitrate, citrate, fumarate, maleate, malate, ascorbate, succinate, tartrate, benzoate, acetate, methanesulfonate, ethanesulfonate, salicylate, stearate, benzenesulfonate or p-toluenesulfonate.

**11.** The use according to any one of claims 6 to 10, wherein the chronic kidney disease is diabetic kidney disease.

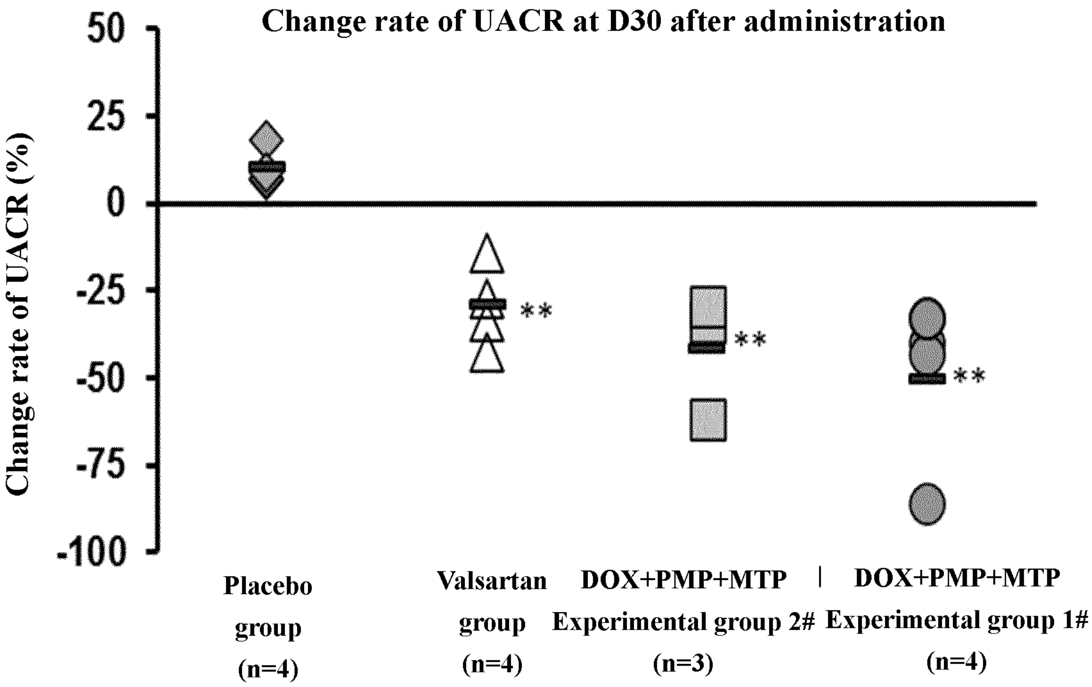
Change rate of UACR at D30 after administration
50
25
0
-25
-50
-75
-100
Change rate of UACR (%)
**
**
**
Placebo group (n=4)
Valsartan group (n=4)
DOX+PMP+MTP Experimental group 2# (n=3)
DOX+PMP+MTP Experimental group 1# (n=4)

Fig. 1

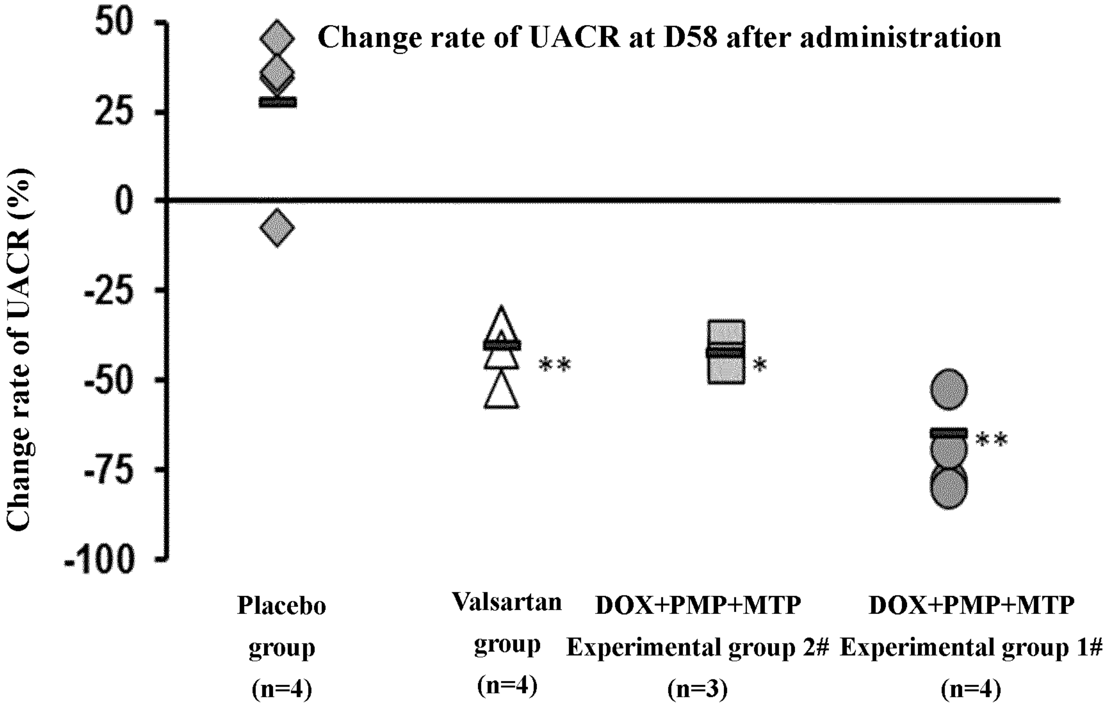
Change rate of UACR at D58 after administration
50
25
0
-25
-50
-75
-100
Change rate of UACR (%)
**
*
**
Placebo group (n=4)
Valsartan group (n=4)
DOX+PMP+MTP Experimental group 2# (n=3)
DOX+PMP+MTP Experimental group 1# (n=4)

Fig. 2

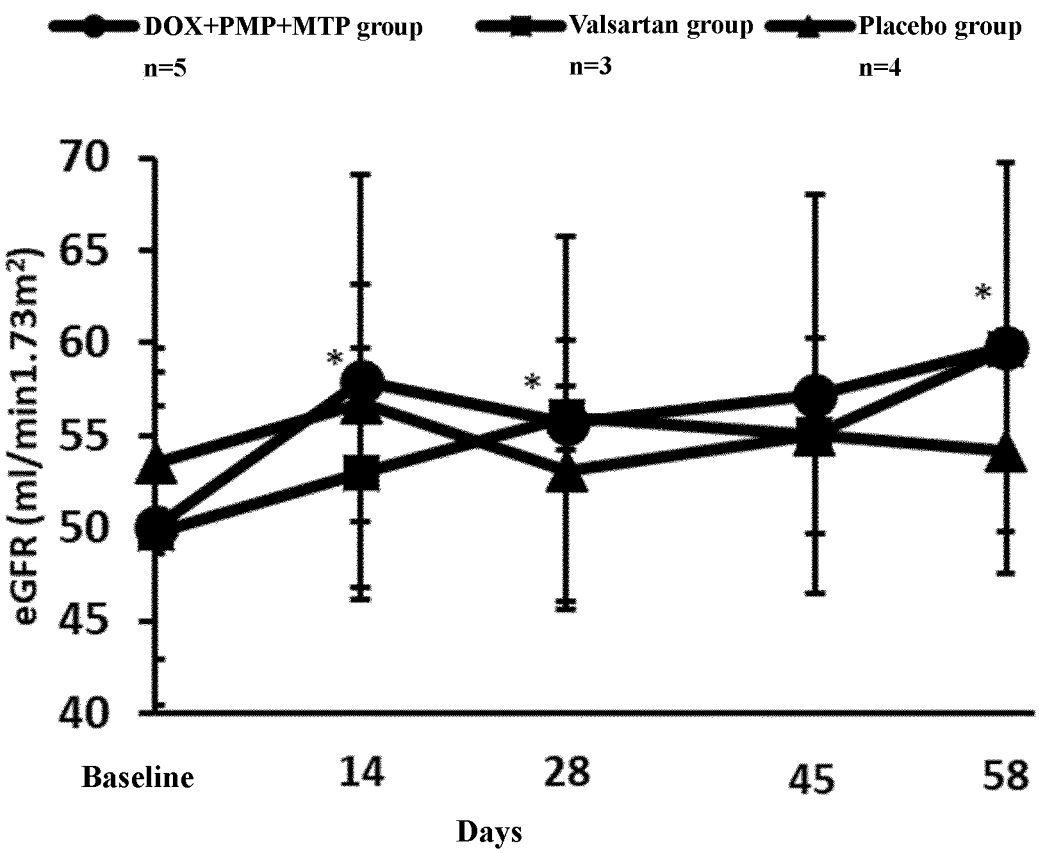
DOX+PMP+MTP group
n=5
Valsartan group
n=3
Placebo group
n=4
70
65
60
55
50
45
40
eGFR (ml/min1.73m²)
*
*
*
Baseline
14
28
45
58
Days

Fig. 3

**INTERNATIONAL SEARCH REPORT**

International application No.
**PCT/CN2021/106355**

**A. CLASSIFICATION OF SUBJECT MATTER**

A61K 31/517(2006.01)i; A61K 31/428(2006.01)i; A61K 31/138(2006.01)i; A61P 13/12(2006.01)i; A61P 3/10(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; CNKI; 万方; ISI Web of Science: 成都文鼎科技发展有限公司, 曾文, 杨真颜, 多沙唑嗪, 可多华, 喹唑嗪, 普拉克索, 米拉帕, 森福罗, 美托洛尔, 立君宁, 倍他乐克, 甲氧乙心安, 美多心安, 美多洛尔, 美他新, 慢性肾病, 糖尿病肾病, CHENGDU WENDING TECHNOLOGY DEVELOPMENT, Zeng Wen, Yang Zhenyan, Supressin, Doxazosin, DOX, Cardura, Pramipexole, Mirapex, Mirapexin, firol, Sifrol, PMP, Metoprolol, MTP, Toprol, Betaloc, Lopresor, Seloken, Chronic Kidney Disease, CKD, DKD, Diabetic Kidney Disease, Diabetic Nephropathy

**C. DOCUMENTS CONSIDERED TO BE RELEVANT**

| Category* | Citation of document, with indication, where appropriate, of the relevant passages | Relevant to claim No. |
|---|---|---|
| A | Marrick L.Kukin et al. "Combined Alpha-Beta Blockade (Doxazosin Plus Metoprolol) Compared With Beta Blockade Alone in Chronic Congestive Heart Failure" *THE AMERICAN JOURNAL OF CARDIOLOGY,* Vol. 77, 01 March 1996 (1996-03-01), pages 486-491, page 490. right-hand column | 1-11 |
| A | US 7348140 B1 (Acadia Pharmaceuticals, Inc.) 25 March 2008 (2008-03-25) entire document | 1-11 |
| A | CA 2610361 A1 (MITSUBISHI TANABE PHARMA CORPORATION) 07 December 2006 (2006-12-07) entire document | 1-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2021** | **28 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/106355**

| Patent document cited in search report | Publication date (day/month/year) | Patent family member(s) | Publication date (day/month/year) |
|---|---|---|---|
| US 7348140 B1 | 25 March 2008 | None | |
| CA 2610361 A1 | 07 December 2006 | CN 101237870 B | 25 May 2011 |
| | | WO 2006129785 A1 | 07 December 2006 |
| | | EP 1894567 B1 | 15 August 2012 |
| | | CN 101237870 A | 06 August 2008 |
| | | US 8754032 B2 | 17 June 2014 |
| | | JP 2013032359 A | 14 February 2013 |
| | | CA 2610361 C | 26 November 2013 |
| | | DK 1894567 T3 | 19 November 2012 |
| | | EP 1894567 A4 | 20 May 2009 |
| | | KR 20130103631 A | 23 September 2013 |
| | | ES 2390231 T3 | 07 November 2012 |
| | | CN 102210865 A | 12 October 2011 |
| | | US 2009082256 A1 | 26 March 2009 |
| | | KR 20080040643 A | 08 May 2008 |
| | | EP 1894567 A1 | 05 March 2008 |
| | | PL 1894567 T3 | 31 January 2013 |
| | | BR PI0611074 A2 | 23 November 2010 |
| | | KR 101438234 B1 | 04 September 2014 |
| | | JP 5586829 B2 | 10 September 2014 |
| | | JP WO2006129785 A1 | 08 January 2009 |
| | | PT 1894567 E | 21 November 2012 |
| | | MX 2007015220 A | 01 March 2008 |
| | | BR 200611074 A2 | 23 November 2010 |
| | | IN 200800022 P4 | 28 November 2008 |
| | | MX 303260 B | 10 September 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DU, Y et al.** *Adrenergic and serotonin receptors affect retinal superoxide generation in diabetic mice: Relationship to capillary degeneration and permeability,* 2015, vol. 29 (5), 2194
- **NAJAFIAN, B. et al.** Glomerulopathy in spontaneously obese rhesus monkeys with type 2 diabetes: a stereological study. *Diabetes Metab Res Rev,* 2011, vol. 27 (4), 341-7
- **LIANG Y ; YANG Z et al.** Diabetic Kidney Disease (DKD) in Nonhuman Primates (NHPs) is Comparable to Humans for Glomerular Filtration Rate (GFR), Histology and High Risk Factors. *ADA,* 2017
- Remington's Pharmaceutical Sciences. Mack Publishing Company, 1990
- **LEVEY AS ; STEVENS LA ; SCHMID CH et al.** A new equation to estimate glomerular filtration rate. *Ann Intern Med,* 2009, vol. 150, 604-612